(19) Europäisches Patentamt
European Patent Office
Office européen des brevets

(11) EP 4 181 874 B1

(12) **EUROPEAN PATENT SPECIFICATION**

(45) Date of publication and mention of the grant of the patent:
**04.06.2025 Bulletin 2025/23**

(21) Application number: **21745844.7**

(22) Date of filing: **12.07.2021**

(51) International Patent Classification (IPC):
***A61K 9/00*** (2006.01) ***A61K 9/06*** (2006.01)
***A61K 47/06*** (2006.01) ***A61K 47/10*** (2017.01)
***A61K 9/107*** (2006.01) ***A61K 31/51*** (2006.01)
***A61P 17/06*** (2006.01) ***A61P 17/04*** (2006.01)
***A61P 17/14*** (2006.01)

(52) Cooperative Patent Classification (CPC):
**A61K 9/0014; A61K 9/06; A61K 9/107; A61K 31/51; A61K 47/06; A61K 47/10; A61P 17/04; A61P 17/06; A61P 17/14**

(86) International application number:
**PCT/IB2021/056232**

(87) International publication number:
**WO 2022/013708 (20.01.2022 Gazette 2022/03)**

(54) **STABLE PHARMACEUTICAL TOPICAL FORMULATION CONTAINING IMMUNOSUPPRESSANT**

STABILE PHARMAZEUTISCHE TOPISCHE FORMULIERUNG ENTHALTEND IMMUNSUPPRESSIVUM

FORMULATION TOPIQUE PHARMACEUTIQUE STABLE CONTENANT UN IMMUNOSUPPRESSEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2020 US 202063053060 P**

(43) Date of publication of application:
**24.05.2023 Bulletin 2023/21**

(73) Proprietor: **Pfizer Inc.**
**New York, NY 10017 (US)**

(72) Inventors:
- **CHEN, Raymond Ruzhong**
**Groton, Connecticut 06340 (US)**
- **SAMUEL, Amanda Patrice Surajhie**
**Cambridge, Massachusetts 02139 (US)**
- **ZELESKY, Todd Christopher**
**Groton, Connecticut 06340 (US)**
- **ZHANG, Xiang**
**Groton, Connecticut 06340 (US)**

(74) Representative: **Potter Clarkson**
**Chapel Quarter**
**Mount Street**
**Nottingham NG1 6HQ (GB)**

(56) References cited:
**WO-A1-2019/040706 US-A1- 2017 296 668**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 181 874 B1

## Description

### FIELD OF THE INVENTION

The present invention relates to a stable topical formulation including an immunosuppressive agent being a JAK-inhibitor, such as ((S)-2,2-difluorocyclopropyl)-((1R,5S)-3-(2-((1-methyl-1H-pyrazol-4-yl)amino)pyrimidin-4-yl)-3,8-diazabicyclo[3.2.1]-octan-8-yl)methanone; ((S)-2,2-difluorocyclopropyl)((1R,5S)-3-(2-((1-propyl-1H-pyrazol-4-yl)amino) pyrimidin-4-yl)-3,8-diazabicyclo[3.21]octan-8-yl)methanone.

### BACKGROUND OF THE INVENTION

Dermatological conditions can be uncomfortable and embarrassing for the patient, so an effective safe treatment is required. Some dermatological conditions are caused by an overactive immune system, including psoriasis, atopic dermatitis, vitiligo, alopecia areata, and can be treated by topical application of immunosuppressive agents, for example topical corticosteroids, calcineurin inhibitors, cyclosporine, and the Phosphodiesterase-4 (PDE4) inhibitor EUCRISA® (crisaborole). More recently, Janus Kinase (JAK) inhibitors have shown clinical efficacy in several dermatologic conditions as topical agents. See, e.g., US Patent No.10,463,675.

((S)-2,2-Difluorocyclopropyl)-((1R,5S)-3-(2-((1-methyl-1H-pyrazol-4-yl)am-ino)-pyrimidin-4-yl)-3,8-diazabicyclo[3.2.1]octan-8-yl)methanone has the chemical formula $C_{18}H_{21}F_2N_7O$ and the following structural formula:

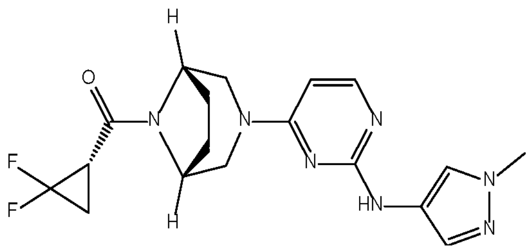

The synthesis of ((S)-2,2-difluorocyclopropyl)-((1R,5S)-3-(2-((1-methyl-1H-pyrazol-4-yl)amino)-pyrimidin-4-yl)-3,8-diazabicyclo[3.2.1]octan-8-yl)methanone is described in commonly assigned US 9,663,526. The crystalline form of ((S)-2,2-difluorocyclopropyl)-((1R,5S)-3-(2-((1-methyl-1H-pyrazol-4-yl)amino)-pyrimid-in-4-yl)-3,8-diazabicyclo [3.2.1]-octan-8-yl)methanone free base, is useful as an inhibitor of protein kinases, such as the enzyme Janus Kinase (JAK) and as such is useful therapeutically as an immunosuppressive agent for organ transplants, xenotransplantation, lupus, multiple sclerosis, rheumatoid arthritis, psoriatic arthritis, inflammatory bowel disease (IBD), psoriasis, Type I diabetes and complications from diabetes, cancer, asthma, atopic dermatitis, autoimmune thyroid disorders, ulcerative colitis, Crohn's disease, Alzheimer's disease, Leukemia and other indications where immunosuppression would be desirable.

Formulations for topical delivery of immunosuppressive agents are commonly creams or ointments with an active pharmaceutical ingredient (API) and one or more excipients. These formulations are typically light or white colored and translucent to opaque in appearance. Squeezable tubes, pumps, bags, or other containers are filled with the immunosuppressive topical formulations and provided with outer paper or cardboard packaging in some cases. The finished and packaged formulations are stored at the manufacturing site, transported to a wholesaler, pharmacy, healthcare clinic, hospital, or healthcare provider and ultimately to the patient or end user who then stores the product during use. This journey from the manufacturing site to the patient can take a substantial amount of time. During the journey, the cream can be subjected to various temperature and humidity conditions.

It is therefore desirable for such topical formulations to be chemically and physically stable over time across a range of storage conditions to provide flexibility to the manufacturing site, intermediaries, and patients in how the product is stored and handled and to ensure product quality is maintained throughout the shelf-life of the product. An important aspect of formulation stability is physical appearance, of which color is one important attribute. Yellowing or darkening of a formulation over time may be perceived as indicating poor product quality.

Heretofore, the inventors believe that this yellowing phenomena or discoloration in such immunosuppressive medicated creams has not been studied adequately or well understood. Commonly, compounds which were believed to enhance chemical stability included antioxidants, such as butyl hydroxyanisole (BHA), butyl hydroxytoluene (BHT), propyl gallate, ascorbic acid (Vitamin C), polyphenols, tocopherols (Vitamin E), and their derivatives were added to excipients and formulations to assist with maintaining a white or lighter color. In fact, some topically applied drug products have in the past added relatively significant amounts of butylated hydroxytoluene (BHT) to maintain a white or lighter color, but still a discoloration problem persists with certain topically applied cream formulations.

Thus, there is a need for stable cream formulations of ((S)-2,2-difluorocyclopropyl)-((1R,5S)-3-(2-((1-methyl-1H-pyrazol-4-yl)amino)pyrimidin-4-yl)-3,8-diazabicyclo[3.2.1]-octan-8-yl)methanone, ((S)-2,2-difluorocyclopropyl) ((1R,5S)-3-(2-((1-propyl-1H-pyrazol-4-yl)amino)pyrimidin-4-yl)-3,8-diazabicyclo[3.2.1]octan-8-yl)methanone, and other JAK-inhibitor topical agents that are resistant to yellowing over time. The present invention provides a novel stable topical formulation characterized by significantly reduced darkening or yellowing.

SUMMARY OF THE INVENTION

According to a first aspect of the invention, according to claim 1, there is provided a topical formulation. comprising an immunosuppressant pharmaceutical agent in an oil-in-water emulsion. The immunosuppressive agent in the instant invention includes JAK inhibitors whose properties are helpful in the treatment of atopic dermatitis, psoriasis, vitiligo, hand eczema, uticaria and other dermatological or autoimmune conditions.

According to the invention, a stable topical formulation is provided comprising a JAK inhibitor in an oil-in-water emulsion, white petrolatum in an amount of 10% by wt.; less than 0.7 ppm butyl hydroxytoluene (BHT) by wt.; oleyl alcohol in an amount of 2% by wt.; and an antimicrobial agent.

In one embodiment, the JAK inhibitor is a JAK1 inhibitor. In another embodiment the JAK inhibitor is a TYK2/JAK1 inhibitor. For example, by way of example and not limitation, the JAK inhibitor comprises ((S)-2,2-difluorocyclopropyl)-((1R,5S)-3-(2-((1-methyl-1H-pyrazol-4-yl)amino)pyrimidin-4-yl)-3,8-diazabicyclo[3.2.1]-octan-8-yl)methanone. In one embodiment, the JAK inhibitor is present in an amount of about 1% to about 3% by wt.

In another embodiment, the formulation includes mineral oil in an amount of about 5% by wt.

In other embodiments, the formulation includes one or more other excipients such as tocopherol in an amount of less than about 1.5 ppm by wt. (for example, added as a stabilizer to the mineral oil in an amount between 0-20 ppm or in some cases to the white petrolatum in an amount of 10 ppm), polyethylene glycol 400 (PEG 400) in an amount of about 10% by wt., diethylene glycol monoethyl ether in an amount of about 15% by wt., emulsifying wax in an amount of about 10% by wt., and the antimicrobial agent comprises 2-phenoxyethanol in an amount of about 1% by wt.

In one embodiment, the formulation maintains a white color having a yellowness index (YI) value of less than about 10 when stored for 24 weeks at 40 degrees C. In another embodiment, the formulation maintains a white color having a yellowness index (YI) value of less than about 6 when stored for 16 weeks at 40 degrees C.

In the invention, yellowing of the cream formulation is minimized by ensuring that the formulation includes not more than 0.7 ppm of BHT. In one embodiment of the invention, yellowing of the cream formulation is minimized by ensuring that the white petrolatum includes not more than 7 ppm of BHT. When the white petrolatum is the sole source of BHT and makes up 10% by wt. of the formulation, this results in not more than 0.7 ppm BHT in the total composition. In other embodiments at least a portion of the not more than 0.7 ppm BHT in the total composition does not come from the white petrolatum, but can come from other ingredients, excipients, or be added independently as an excipient.

While developing and optimizing various formulations of such immunosuppressive agents, including but not limited to a JAK1 modulator, more specifically a TYK2/JAK1 modulator, in a topical cream for the treatment of atopic dermatitis and psoriasis, the present inventors have observed that the drug product, which initially appears white upon manufacture, develops a pale-yellow color that increases in intensity with increasing (a) active pharmaceutical ingredient (API) concentration, (b) time, and (c) storage temperature. The present inventors have surprisingly and unexpectedly identified the excipients that drive the cream's color change from white to pale-yellow. More particularly, the present inventors have discovered that the concentration of BHT (butylated hydroxytoluene) in the formulation has a statistically significant quadratic effect on yellowing for samples stored at 25°C, 30°C, and 40°C, and as a result, have determined certain critical range limits for the amount of BHT in the drug product which minimize yellow color development.

In a preferred embodiment, the invention provides a formulation which uses a BHT-free white petroleum stabilized with 10 ppm tocopherol and includes oleyl alcohol to minimize yellow color development. In other embodiments, the invention provides other formulations which result in acceptable color development, wherein the total cream composition contains 0.7 ppm BHT or less, corresponding to a concentration of BHT in white petrolatum of not more than 7 ppm when the white petrolatum is about 10% by wt. and the petrolatum is the only source of BHT. Additionally, the invention surprisingly and unexpectedly provides formulations wherein a variety of excipient combinations are acceptable for product purity so long as the BHT limitations are maintained, whereby no degradants are observed in formulations stored at 40°C for 24 weeks.

The term "% by weight" used herein refers to the % by weight in the final formulation. In other words, if the final formulation weighs 100 grams and an ingredient is 10% by weight or 10% wt. or 10% w/w, then 10 grams of the ingredient is present in the final formulation. In the case of an ingredient described in parts per million, 1 ppm is 0.0001 wt. %.

The term "ppm" used herein means parts per million.

The term "storage temperature" used herein is defined as the ambient temperature in the chamber in which the samples were stored in their primary containers - either a sealed glass vial or a sealed foil laminate tube. Relative humidity was controlled as is standard in the art. Similarly, normal atmospheric pressure conditions were utilized. Temperatures

provided herein are storage temperatures unless stated otherwise and are expressed in degrees Celsius, which is also abbreviated herein as °C.

The term "mineral oil" as used herein means a transparent, odorless liquid comprising a mixture of refined saturated aliphatic and cyclic hydrocarbons obtained from petroleum. The term mineral oil includes light mineral oil (liquid paraffin) and regular or higher specific gravity or density mineral oil.

The term "white petrolatum" is commonly understood in the pharmaceutical field in the United States and is also known as soft white paraffin in other regions of the world.

The term "about" is +/- 15% whether referring to wt. % or ppm values stated herein. For example, about 10% wt. should be understood to encompass a range of values from 8.5% to 11.5% and 10 ppm should be understood to encompass a range of values from 8.5 ppm to 11.5 ppm.

The term "oil-in water emulsion" as used herein means a semi-solid mixture comprising two immiscible phases, an oil phase and an aqueous phase. Oil phase droplets are suspended in the aqueous phase and the emulsion is stabilized using an emulsifying agent.

One advantage of the present invention is that it provides a white or light-colored topical delivery formulation for an immunosuppressive agent that is more resistant to yellowing, and does not yellow as much nor as quickly as the previously disclosed formulation.

This and other advantages will be apparent to one skilled in the art from the drawings and the description that follow.

## BRIEF DESCRIPTION OF THE FIGURES

The invention will now be described, by way of illustration only, with reference to the following examples, tables and figures accompanying the specification.

FIG. 1 is a graphical representation of Yellowness Index (YI(E313-96)(D65)) of 28 cream formulation samples measured in glass vials (YI-Vial) over time from an initial time $t_0$ to 24 weeks under various storage temperatures. The figure shows Time (in weeks) on the lower X-axis and YI-Vial on the left-hand Y axis. The graphs of the individual sample runs are grouped according to increasing Storage Temperature in °C (5, 25, 30, and 40) as seen in the upper X-axis of the figure.

FIG. 2 is a contour plot of Yellowness Index (YI-Cuvette) at 24 weeks at 5°C storage temperature with one embodiment of the formulation of the present invention with the excipient combination of Calumet™ White Petrolatum, BASF™ Oleyl Alcohol, and Dow™ PEG 400, which was the best-case scenario at that storage temperature. Added Tocopherol is shown on the X-axis in parts per million (ppm) and Added BHT is shown on the Y-axis in parts per million (ppm). Contoured lines of constant YI-Cuvette are plotted and labeled.

FIG. 3 is a contour plot of Yellowness Index (YI-Cuvette) at 24 weeks at 5°C storage temperature with a less advantageous alternative embodiment of the formulation of the present invention with the excipient combination of Calumet™ White Petrolatum, Croda™ Oleyl Alcohol, and Croda™ PEG 400, which was the worst-case scenario at that storage temperature. Added Tocopherol is shown on the X-axis in ppm and Added BHT is shown on the Y-axis in ppm. Contoured lines of constant YI-Cuvette are plotted and labeled.

FIG. 4 is contour plot of Yellowness Index (YI-Cuvette) at 24 weeks at 25°C storage temperature with another embodiment of the formulation of the present invention with the excipient combination of Croda™ White Petrolatum, Croda™ Oleyl Alcohol, and Dow™ PEG 400, which was the best-case scenario at that storage temperature. Added Tocopherol is shown on the X-axis in ppm and Added BHT is shown on the Y-axis in ppm. Contoured lines of constant YI-Cuvette are plotted and labeled.

FIG. 5 is a contour plot of Yellowness Index (YI-Cuvette) at 24 weeks at 25°C storage temperature with a less advantageous alternative embodiment of the invention with the excipient combination of Calumet™ White Petrolatum, Croda™ Oleyl Alcohol, and Croda™ PEG 400, which was the worst-case scenario at that storage temperature. Added Tocopherol is shown on the X-axis in ppm and Added BHT is shown on the Y-axis in ppm. Contoured lines of constant YI-Cuvette are plotted and labeled.

FIG. 6 is a contour plot of Yellowness Index (YI-Cuvette) at 24 weeks at 30°C storage temperature with another embodiment of the invention with the excipient combination of Croda™ White Petrolatum, Croda™ Oleyl Alcohol, and Dow™ PEG 400, which was the best-case scenario at that storage temperature. Added Tocopherol is shown on the X-axis in ppm and Added BHT is shown on the Y-axis in ppm. Contoured lines of constant YI-Cuvette are plotted and labeled.

FIG. 7 is a contour plot of Yellowness Index (YI-Cuvette) at 24 weeks at 30°C storage temperature with a less advantageous alternative embodiment of the invention with the excipient combination of Calumet™ White Petrolatum, Croda™ Oleyl Alcohol, and Croda™ PEG 400, which was the worst-case scenario at that storage temperature. Added Tocopherol is shown on the X-axis in ppm and Added BHT is shown on the Y-axis in ppm. Contoured lines of constant

YI-Cuvette are plotted and labeled.

FIG. 8 is a contour plot of Yellowness Index (YI-Cuvette) at 24 weeks at 40°C storage temperature with another embodiment of the invention with the excipient combination of Calumet™ White Petrolatum, BASF™ Oleyl Alcohol, and Dow™ PEG 400, which was the best-case scenario at that storage temperature. Added Tocopherol is shown on the X-axis in ppm and Added BHT is shown on the Y-axis in ppm. Contoured lines of constant YI-Cuvette are plotted and labeled.

FIG. 8A is an enlarged or zoomed-in contour plot of YI-Cuvette taken from the lower left region of FIG. 8 at 24 weeks at 40°C storage temperature. Added Tocopherol is shown on the X-axis in ppm and Added BHT is shown on the Y-axis in ppm. Contoured lines of constant YI-Cuvette are plotted and labeled.

FIG. 9 is a contour plot of Yellowness Index (YI-Cuvette) at 24 weeks at 40°C storage temperature with a less advantageous alternative embodiment of the invention with the excipient combination of Calumet™ White Petrolatum, Croda™ Oleyl Alcohol, and Croda™ PEG 400, which was the worst-case scenario at that storage temperature. Added Tocopherol is shown on the X-axis in ppm and Added BHT is shown on the Y-axis in ppm. Contoured lines of constant YI-Cuvette are plotted and labeled.

FIG. 10 is a graph of Yellowness Index (YI(E313-96)(D65)) or YI-Cuvette on the Y-axis versus storage time in weeks on the X-axis for various cream formulations including 1% w/w API stored at 40°C from initial time $t_0$ to 16 weeks.

FIG. 11 is a graph of Yellowness Index (YI(E313-96)(D65)) or YI-Cuvette on the Y-axis versus storage time in weeks on the X-axis for various cream formulations including 3% w/w API stored at 40°C from initial time $t_0$ to 16 weeks.

## DETAILED DESCRIPTION OF THE INVENTION

The present invention relates to a stable formulation of compounds which are JAK inhibitors useful for the treatment of diseases and conditions associated with dysregulation of JAK, in particular JAK1 and TYK2/JAK1. The present invention further provides pharmaceutical compositions comprising such JAK inhibitors useful for treating and/or preventing such diseases and conditions.

According to the invention there is provided a topical formulation comprising:

a JAK inhibitor in an oil-in-water emulsion;
white petrolatum in an amount of 10% by wt.;
less than 0.7 ppm butyl hydroxytoluene (BHT) by wt.;
oleyl alcohol in an amount of 2% by wt.; and
an antimicrobial agent.

Suitably in some embodiments, the JAK inhibitor is a JAK1 inhibitor.

Suitably in some embodiments, the JAK inhibitor is a TYK2/JAK1 inhibitor.

Suitably in some embodiments, the JAK inhibitor is ((S)-2,2-difluorocyclopropyl)-((1R,5S)-3-(2-((1-methyl-1H-pyrazol-4-yl)amino)pyrimidin-4-yl)-3,8-diazabicyclo[3.2.1]-octan-8-yl)methanone.

Suitably in some embodiments, the JAK inhibitor is present in an amount of about 1% to about 3% by wt.

Suitably in some embodiments, the topical formulation further comprises tocopherol in an amount of less than about 6.5 ppm.

Suitably in some embodiments, the topical formulation further comprises polyethylene glycol 400 in an amount of about 10% by wt.

Suitably in some embodiments, the topical formulation further comprises diethylene glycol monoethyl ether in an amount of about 15% by wt.

Suitably in some embodiments, the topical formulation further comprises an emulsifying wax in an amount of about 10% by wt.

Suitably in some embodiments, the antimicrobial agent comprises 2-phenoxyethanol in an amount of about 1% by wt.

Suitably in some embodiments, the topical formulation maintains a white color having a yellowness index (YI) value of less than about 10 when stored for 24 weeks at 40 degrees C.

Suitably in some embodiments, the topical formulation maintains a white color having a yellowness index value of less than about 6 when stored for 16 weeks at 40 degrees C.

Suitably in some embodiments, the topical formulation has a yellowness index value that increases by not more than about 8 over 24 weeks at 40 degrees C.

Suitably in some embodiments, the white petrolatum includes not more than 7 ppm of BHT.

Suitably in some embodiments, at least a portion of the 0.7 ppm BHT does not come from the white petrolatum.

Suitably in some embodiments, the topical formulation further comprises mineral oil in an amount of about 5% by wt.

Suitably in some embodiments, there is provided a topical formulation for use in the treatment of a dermatological condition comprising:

a JAK inhibitor as an oil in purified water emulsion;
white petrolatum in an amount of 10% by wt.;
tocopherol in an amount of less than about 6.5 ppm;
oleyl alcohol in an amount of 2% by wt.;
polyethylene glycol 400 in an amount of about 10% by wt.;
diethylene glycol monoethyl ether in an amount of about 15% by wt.;
mineral oil in an amount of about 5% by wt.;
an emulsifying wax in an amount of about 10% by wt.; and
an antimicrobial agent comprises 2-phenoxyethanol in an amount of about 1% by wt.;
wherein the formulation is free of BHT, i.e. less than 0.7 ppm by wt., and thereby maintains a yellowness index of less than about 6 when stored at 40 degrees C for 16 weeks and not more than about 10 when stored for 24 weeks at 40 degrees C.

Suitably in some embodiments, there is provided a topical formulation comprising: a compound having the structural formula:

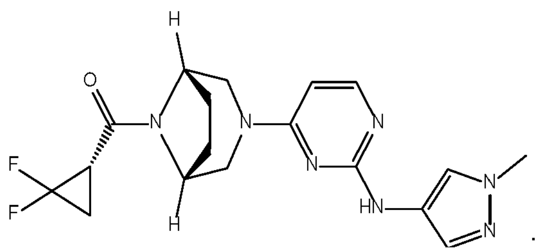

white petrolatum in an amount of 10% by wt. and having tocopherol in an amount of less than about 10 ppm;
less than 0.7 ppm butyl hydroxytoluene (BHT) by wt.;
oleyl alcohol in an amount of 2% by wt.;
polyethylene glycol 400 in an amount of about 10% by wt.;
diethylene glycol monoethyl ether in an amount of about 15% by wt.;
mineral oil in an amount of about 5% by wt.;
an emulsifying wax in an amount of about 10% by wt.; and
an antimicrobial agent.

Suitably in some embodiments, there is provided a topical formulation of the present invention for use in treating or preventing a disease or condition selected from psoriasis, atopic dermatitis, atopic eczema, chronic hand eczema, uticaria, vitiligo, cutaneous lupus and alopecia areata.

Suitably in some embodiments, the topical formulation is administered at least once daily.

Suitably in some embodiments, the disease or condition is psoriasis.

Suitably in some embodiments, the disease or condition is atopic dermatitis.

Suitably in some embodiments, the disease or condition is hand eczema.

Suitably in some embodiments, the disease or condition is uticaria.

Suitably in some embodiments, the disease or condition is cutaneous lupus.

Suitably in some embodiments, there is provided a topical formulation of the present invention for use in the treatment of a disorder for which a TYK2/JAK1 inhibitor is indicated.

The invention discloses a formulation for a topical dose form. Compositions for topical administration also include, for example, topical gels, sprays, ointments, and creams. A topical formulation may include a crystalline or liquid compound which enhances absorption or penetration of the active ingredient through the skin or other affected areas. Typical formulations for this purpose include gels, hydrogels, lotions, solutions, creams, ointments, dusting powders, dressings, foams, films, skin patches, wafers, implants, sponges, fibres, bandages and microemulsions. Typical excipients include alcohol, water, mineral oil, liquid petrolatum, white petrolatum, glycerin, polyethylene glycol, propylene glycol, and dietheylene glycol monoethyl ether. Penetration enhancers may be incorporated - see, for example, B. C. Finnin and T. M. Morgan, J. Pharm. Sci., vol. 88, pp. 955-958, 1999.

Accordingly, topical formulations of the presently disclosed compound of ((S)-2,2-difluorocyclopropyl) ((1R,5S)-3-(2-((1-methyl-1H-pyrazol-4-yl)amino)pyrimidin-4-yl)-3,8-diazabicyclo[3.2.1]octan-8-yl)methanone, which is

a JAK inhibitor or more particularly a JAK1 inhibitor or even more particularly a TYK2/JAK1 inhibitor, may be administered using such preparations encompassing all conventional methods of administration across the surface of the body and the inner linings of body passages including epithelial and mucosal tissues, including transdermal, epidermal, buccal, pulmonary, ophthalmic, intranasal, vaginal and rectal modes of administration.

Topical formulations containing ((S)-2,2-difluorocyclopropyl)-((1R,5S)-3-(2-((1-methyl-1H-pyrazol-4-yl)amino) pyrimidin-4-yl)-3,8-diazabicyclo[3.2.1]octan-8-yl)methanone can be given in therapeutically effective amounts in daily or twice daily doses to patients in need. These amounts range from about 0.1% to about 5.0% wt., more preferably, from about 0.1% to about 3.0% wt.

While working on the development, stability and improvement of the above-mentioned TYK2/JAK1 topical formulation and other similar compounds, the inventors studied the effects over time of both API concentration and storage temperature on the stability of the formulation. The inventors observed discoloration effects over time of formulations containing active pharmaceutical ingredient at relatively low concentration levels. When samples of the formulation were stored in filled foil laminate tubes for three (3) months at room temperature of about 25°C and then sliced open along a major central vertical plane, it was observed that the tubes filled with just a vehicle, 0% or no API, exhibited little if any discoloration. With 0.1% API, some slight yellowing occurred. With 0.3% API, the discoloration increased slightly. With more moderate and typical levels of API, such as for example 1%, the yellowing was more noticeable, and with 3% API was even more noticeable.

The inventors also studied the effects over time of different storage temperatures by storing and then bisecting sample-filled foil laminate tubes as follows: 0% API at 5°C for six (6) months; and 3% API at 5°C, 25°C, 30°C for six (6) months respectively and 40°C for three (3) months. The yellowing noticeably increased with increased storage temperature for the 5°C, 25°C, 30°C samples including 3% API after they were stored for six (6) months. The 40°C/3% API sample was only stored for three (3) months before being cut open and it was still noticeably darker or yellower than any of the other samples that had been stored longer at lower temperatures.

Because the API% is often driven by efficacy, safety, and other considerations, the inventors choose to focus on 1%-3% API for further experiments to understand which ingredients or excipients were contributing to the yellowing phenomena. A storage temperature of 5°C seemed to provide a reasonable base case because little discoloration was observed. The storage temperatures of 25°C, 30°C and 40°C seemed to produce easily observable and thus measurable yellowness variations to facilitate further study of the yellowing phenomena by the inventors. Regulatory authorities may request data for storage temperatures of 5°C, 25°C, and 30°C along with a new drug application, and 40 °C is a useful temperature for production of accelerated aging data.

Like others attempting to formulate safe, effective, and stable medicated topical creams, the inventors had a multitude of potential ingredients, stabilizers and excipients to choose from and each of those items were commercially available from a number of different sources, who sometimes had their own proprietary subset of ingredients, stabilizers and excipients. This means the possible combinations were practically infinite and to explore them all normally would take years and undue experimentation without a better understanding of the key drivers of the discoloration. Of course, as described above, certain APIs can present their own unique challenges in achieving effective and stable formulations. Without limiting the invention, the following exemplary ingredients, stabilizers and excipients were studied for use with JAK1 and TYK2/JAK1 topical creams.

1. White petrolatum sold under the trademarks:

   a. Calumet Super White™ Petrolatum USP, which can contain up to 20 ppm BHT and is available from Calumet Refining, Indianapolis, IN, USA (hereinafter Calumet™ more generally); and
   b. Crolatum™ V-SO, which contains 10 ppm tocopherol and is available from Croda International, Snaith, East Riding of Yorkshire, UK (hereinafter Croda™ more generally).

2. Oleyl alcohol sold under the trademarks:

   a. Kollicream™ OA, which contains 250 ppm CONTROX® K SC and is available from BASF, Ludwigshafen, GERMANY (hereinafter BASF™ more generally); and
   b. Super Refined™ Novol™ by Croda International, Snaith, East Riding of Yorkshire, UK (hereinafter Croda™ more generally).

3. Polyethylene glycol 400 (PEG 400) sold under the trademarks:

   a. CARBOWAX™ by Dow Chemical Company, Midland, MI, USA (hereinafter Dow™ more generally); and
   b. Croda™ Super Refined PEG 400 by Croda International, Snaith, East Riding of Yorkshire, UK (hereinafter Croda™ more generally).

4. Butyl hydroxytoluene (BHT) sold under the name Butylated Hydroxytolune, Granular, NF by Spectrum Chemical Manufacturing Corporation, New Brunswick, NJ, USA.
5. DL-α-Tocopherol sold by Alfa Aesar by Thermo Fisher Scientific, 2 Radcliff, Rd., Tewksbury, MA 01876, USA.
6. Highly purified Diethylene glycol monoethyl ether, EP/NF, sold under the trademark Transcutol™ HP by GATTEFOSSE SAS, 36 Chemin de Genas 69800 Saint-Priest, FRANCE (hereinafter Gattefosse™ more generally).
7. Emulsifying Wax, NF sold by Spectrum Chemical Manufacturing Corporation, New Brunswick, NJ, USA (hereinafter Spectrum™ more generally).
8. Mineral oil sold under the trademark Drakeol™ 32 by Calumet Refining, Indianapolis, IN, USA, which can contain up to 20 ppm Tocopherol (hereinafter Calumet™ more generally).
9. An antimicrobial agent, such as phenoxyethanol or more particularly 2-phenoxyethanol sold under the name 2-phenoxyethanol multi-compendial A&C grade by A&C American Chemicals Ltd., 3010 Rue De Baene, Montreal, QC H4S 1L2, CANADA (hereinafter more generally A&C™).

With respect to a JAK inhibitor, more particularly a JAK1 inhibitor, and even more particularly a TYK2/JAK1 inhibitor, this disclosure provides the results and statistical conclusions for topical cream excipient experiments wherein a Yellowness Index per ASTM (American Society for Testing and Materials) Method E313, YI(E313-96)(D65) was measured in a cuvette ("YI-Cuvette") for each sample at 24 weeks at several storage temperatures (5°C, 25°C, 30°C and 40°C). This design of experiment (DoE) employed a 28-run D-optimal statistical design to evaluate the effects of five factors: Added BHT (0, 15, 30 ppm), Added Tocopherol (0, 15, 30 ppm), White Petrolatum (Calumet™, Croda™), Oleyl Alcohol (BASF™, Croda™), and PEG 400 (Dow™, Croda™) on the yellowness of the cream. Higher YI(E313-96)(D65) values indicate yellower cream. During the experiment, YI(E313-96)(D65) was measured in a standard glass vial ("YI-Vial") from initial time $t_0$ to 24 weeks (0, 2, 4, 6, 8, 10, 12, 24 weeks) as shown in FIG. 1 and was measured in a cuvette ("YI-Cuvette") at 24 weeks. Therefore, YI-Cuvette at 24 weeks at those four different storage temperatures are the DoE responses analyzed and presented in this disclosure. The goal of the experiment was to identify the excipient factors, levels, and compositions which would minimize the yellowness index. With the fitted DoE models, the predicted means of YI-Cuvette at 24 weeks at 40°C could be used to select the practical color-minimizing excipient factors, levels, and compositions. The statistical analysis results are summarized below.

1) At 5°C, YI-Vial maintained a low level from initial to 24 weeks. At 24 weeks, YI-Cuvette was below 0 for all the 28 runs. Added Tocopherol had a statistically significant quadratic effect on YI-Cuvette at 24 weeks. Added BHT had statistically significant two-way interaction effects on YI-Cuvette at 24 weeks with White Petrolatum and PEG 400.
2) At 25°C, Added BHT and Added Tocopherol had statistically significant quadratic and two-way interaction effects on YI-Cuvette at 24 weeks. There was also a statistically significant main effect of PEG 400 and a weakly statistically significant ($p = 0.0538$) two-way interaction effect of White Petrolatum and Oleyl Alcohol.
3) At 30°C, Added BHT and Added Tocopherol had statistically significant quadratic and two-way interaction effects on YI-Cuvette at 24 weeks. There were also statistically significant two-way interaction effects of Added BHT and White Petrolatum, Added Tocopherol and PEG 400, and White Petrolatum and Oleyl Alcohol.
4) At 40°C, there was a statistically significant quadratic effect of Added BHT, and main effects of Added Tocopherol and PEG 400.

Based on the fitted DoE models **(Table A3),** the predicted means (and the upper limits of their 95% confidence intervals) of YI-Cuvette at 24 weeks at different storage temperatures for all the eight excipient combinations with 0 ppm Added Tocopherol (best-case in terms of Added Tocopherol level) are provided in **Table 0** below.

**Table 0. The Predicted Means of YI-Cuvette at 24 Weeks with 0 ppm Added Tocopherol**

| Excipient Comb. ID | White Petrolatum | Oleyl Alcohol | PEG 400 | Added BHT (ppm) | Predicted Mean (Upper Limit of 95% Confidence Interval) of YI-Cuvette at 24 Weeks | | | |
|---|---|---|---|---|---|---|---|---|
| | | | | | 5°C | 25°C | 30°C | 40°C |
| 1 | Calumet™ | BASF™ | Dow™ | 0 | -8.58 (-8.27) | -0.22 (1.00) | 1.18 (2.01) | 4.80 (5.81) |
| | | | | 1.5 | -8.50 (-8.18) | 0.50 (1.94) | 2.30 (3.36) | 6.20 (7.41) |

(continued)

| Excipient Comb. ID | White Petrolatum | Oleyl Alcohol | PEG 400 | Added BHT (ppm) | Predicted Mean (Upper Limit of 95% Confidence Interval) of YI-Cuvette at 24 Weeks | | | |
|---|---|---|---|---|---|---|---|---|
| | | | | | 5°C | 25°C | 30°C | 40°C |
| 2 | Calumet™ | BASF™ | Croda™ | 0 | -7.72 (-7.21) | 1.02 (2.84) | 4.17 (5.92) | 6.21 (7.52) |
| | | | | 1.5 | -7.67 (-7.18) | 2.08 (4.13) | 6.14 (8.12) | 8.03 (9.63) |
| 3 | Calumet™ | Croda™ | Dow™ | 0 | -7.74 (-7.28) | 1.13 (2.80) | 2.64 (3.85) | 4.80 (5.81) |
| | | | | 1.5 | -7.68 (-7.24) | 2.21 (4.12) | 4.24 (5.70) | 6.20 (7.41) |
| **4** | **Calumet™** | **Croda™** | **Croda™** | **0** | **-6.23 (-5.38)** | **2.94 (5.23)** | **6.69 (8.75)** | **6.21 (7.52)** |
| | | | | **1.5** | **-6.29 (-5.49)** | **4.36 (6.91)** | **9.14 (11.44)** | **8.03 (9.63)** |
| **5** | **Croda™** | **BASF™** | **Dow™** | **0** | **-8.58 (-8.27)** | **-0.37 (0.86)** | **0.26 (0.77)** | **4.80 (5.81)** |
| 6 | Croda™ | BASF™ | Croda™ | 0 | -7.72 (-7.21) | 0.79 (2.53) | 2.05 (3.23) | 6.21 (7.52) |
| **7** | **Croda™** | **Croda™** | **Dow™** | **0** | **-7.74 (-7.28)** | **-0.61 (0.35)** | **0.22 (0.64)** | **4.80 (5.81)** |
| 8 | Croda™ | Croda™ | Croda™ | 0 | -6.23 (-5.38) | 0.39 (1.83) | 1.89 (2.92) | 6.21 (7.52) |

The primary objective of the topical cream excipient DoE was to identify the excipients and their interactions, which drove the cream's color change from white to yellow. Among the five excipient factors identified to have potential effects on the color change, Added BHT and Added Tocopherol (Note: the amounts added were relative to the total formulation weight) were continuous numeric factors, while White Petrolatum, Oleyl Alcohol and PEG 400 were two-level categoric factors whose levels were different excipient sources. Besides the studied excipients, Gattefosse™ Transcutol™ HP, Spectrum™ emulsifying wax, Calumet™ mineral oil and A&C™ Phenoxyethanol were used for all the experimental runs.

All cream samples were manufactured using the following general procedure. Water, PEG 400, and 2-phenoxyethanol were combined in the main mixing vessel and heated to approximately 60°C (aqueous phase). In a separate vessel, mineral oil, white petrolatum, oleyl alcohol and emulsifying wax were heated to 60°C while mixing until melted (oil phase). The oil phase mixture was added to the main mixing vessel containing the aqueous phase mixture while homogenizing. The resulting cream was cooled to approximately 35°C while homogenizing. The API was dissolved in diethylene glycol monoethyl ether at room temperature. The API solution was added to the main mixing tank while homogenizing. The resulting active cream was cooled to room temperature and filled into glass vials or foil laminate tubes.

The cream samples for each experimental run had active pharmaceutical ingredient (API) concentration of 30mg/g (3% by wt.) and were stored in sealed glass vials at 5°C, 25°C, 30°C and 40°C storage temperatures. Bi-weekly to monthly color measurements including the Yellowness Index YI(E313-96)(D65) were taken up to 24 weeks. Higher YI(E313-96)(D65) values indicate yellower cream. During the experiment, YI(E313-96)(D65) was measured directly in the sealed glass vials (YI-Vial) from the start or initial time or $t_0$ to 24 weeks (0, 2, 4, 6, 8, 10, 12, 24 weeks). Additionally, samples were removed from the glass vials at 24 weeks and transferred to disposable plastic cuvettes for YI(E313-96)(D65) measurements, which are referred to herein as YI-Cuvette. The cuvette measurement method was used by the project team moving forward, therefore, YI(E313-96)(D65) measured in the cuvette (YI-Cuvette) at 24 weeks at those four different storage temperatures are the DoE responses analyzed and presented in this disclosure. With the fitted DoE models, the predicted means of YI-Cuvette at 24 weeks at 40°C could be used to select the practical color-minimizing excipient compositions.

As mentioned above, the topical cream excipient DoE employed a 28-run D-optimal statistical design to assess the main and two-way interaction effects of the five excipient factors and the quadratic effects of Added BHT and Added

Tocopherol. **Table 1** provides the statistical design and factor levels of the experiment.

In the design of experiments, a main effect is the difference between levels of an independent variable on a dependent variable averaged across the levels of any other independent variables. A two-way interaction effect occurs when the effect of an independent variable on a dependent variable depends on the level of another independent variable. A quadratic effect is an interaction term where an independent variable interacts with itself. A statistically significant effect indicates that the effect is statistically significantly different from 0.

**Table 1. Experimental Design of Topical Cream Excipient DoE**

| Run | Factor A: Added BHT (unit: ppm) | Factor B: Added Tocopherol (unit: ppm) | Factor C: White Petrolatum | Factor D: Oleyl Alcohol | Factor E: PEG 400 |
|---|---|---|---|---|---|
| 1 | 0 | 30 | Croda™ | BASF™ | Croda™ |
| 2 | 0 | 30 | Calumet™ | BASF™ | Croda™ |
| 3 | 0 | 15 | Calumet™ | BASF™ | Dow™ |
| 4 | 0 | 30 | Calumet™ | Croda™ | Croda™ |
| 5 | 0 | 0 | Calumet™ | Croda™ | Dow™ |
| 6 | 15 | 0 | Croda™ | BASF™ | Croda™ |
| 7 | 30 | 30 | Calumet™ | Croda™ | Dow™ |
| 8 | 30 | 30 | Croda™ | Croda™ | Croda™ |
| 9 | 0 | 30 | Croda™ | Croda™ | Dow™ |
| 10 | 30 | 30 | Croda™ | Croda™ | Croda™ |
| 11 | 0 | 0 | Calumet™ | Croda™ | Dow™ |
| 12 | 30 | 0 | Calumet™ | BASF™ | Dow™ |
| 13 | 30 | 0 | Croda™ | Croda™ | Dow™ |
| 14 | 0 | 0 | Calumet™ | BASF™ | Croda™ |
| 15 | 0 | 0 | Croda™ | BASF™ | Dow™ |
| 16 | 30 | 30 | Croda™ | BASF™ | Dow™ |
| 17 | 15 | 0 | Calumet™ | BASF™ | Dow™ |
| 18 | 15 | 15 | Croda™ | BASF™ | Dow™ |
| 19 | 0 | 30 | Croda™ | Croda™ | Dow™ |
| 20 | 15 | 15 | Calumet™ | Croda™ | Croda™ |
| 21 | 0 | 0 | Croda™ | Croda™ | Croda™ |
| 22 | 30 | 30 | Calumet™ | Croda™ | Dow™ |
| 23 | 0 | 0 | Croda™ | Croda™ | Croda™ |
| 24 | 15 | 30 | Calumet™ | BASF™ | Dow™ |
| 25 | 30 | 30 | Calumet™ | BASF™ | Croda™ |
| 26 | 30 | 15 | Croda™ | BASF™ | Croda™ |
| 27 | 30 | 0 | Calumet™ | Croda™ | Croda™ |
| 28 | 30 | 0 | Croda™ | BASF™ | Croda™ |

The detailed data, which contained factor levels and responses, was stored in an Excel™ file for subsequent analysis. The data visualization and statistical analysis were performed in SAS JMP® 14.0.0 and Design-Expert® (v11.0.6.0).

Yellowness Indexes YI(E313-96)(D65) measured for samples in vials (YI-Vial) from initial time $t_0$ to 24 weeks (0, 2, 4, 6, 8 10,12, 24 weeks) are recorded in Tables D5, D25, D30, and D40 below.

**Table D5. Yellowness Indexes measured in vials (YI-Vial) for samples stored at 5°C**

| Run | 0 weeks | 2 weeks | 4 weeks | 6 weeks | 8 weeks | 10 weeks | 12 weeks | 24 weeks |
|---|---|---|---|---|---|---|---|---|
| 1 | -1.12 | -1.85 | -1.80 | -1.59 | -1.28 | -1.25 | -1.7 | -2.25 |
| 2 | -1.51 | -2.13 | -2.15 | -1.84 | -1.64 | -1.41 | -0.33 | -0.46 |
| 3 | -1.49 | -1.62 | -1.7 | -1.83 | -1.02 | -1.24 | -1.52 | -1.71 |
| 4 | -0.19 | -0.78 | -1.17 | -1.13 | -0.72 | -0.62 | 0.21 | -0.03 |
| 5 | -0.89 | -1.33 | -1.36 | -1.63 | -1.08 | -1.30 | -1.21 | -2.16 |
| 6 | -1.55 | -2.17 | -2.34 | -1.93 | -1.9 | -1.65 | -1.53 | -2.68 |
| 7 | -0.88 | -1.53 | -1.44 | -1.35 | -0.91 | -0.95 | -0.05 | 0.69 |
| 8 | -1.54 | -2.38 | -2.48 | -2.47 | -2.24 | -1.68 | -2.40 | -1.85 |
| 9 | -0.37 | -0.90 | -0.99 | -1.00 | -1.08 | -0.83 | -1.19 | -1.89 |
| 10 | -0.94 | -1.36 | -1.42 | -1.57 | -0.62 | -0.84 | -1.19 | -1.66 |
| 11 | -0.82 | -1.54 | -1.51 | -1.85 | -1.45 | -1.88 | -1.53 | -2.59 |
| 12 | -0.82 | -1.10 | -1.21 | -1.29 | -0.58 | -0.52 | -0.97 | -2.13 |
| 13 | -0.78 | -1.18 | -1.33 | -1.35 | -1.17 | -1.11 | -1.74 | -1.89 |
| 14 | -0.97 | -1.49 | -1.61 | -1.60 | -1.63 | -1.68 | -1.66 | -2.12 |
| 15 | -1.77 | -1.85 | -1.93 | -1.83 | -1.57 | -1.35 | -2.47 | -2.98 |
| 16 | -0.58 | -0.90 | -0.94 | -0.66 | -0.59 | -0.25 | 0.56 | 1.27 |
| 17 | -1.44 | -1.50 | -1.44 | -1.59 | -1.21 | -1.15 | -1.36 | -1.69 |
| 18 | -0.94 | -1.16 | -1.19 | -1.01 | -0.63 | -0.42 | -0.42 | -0.91 |
| 19 | -0.72 | -1.33 | -1.6 | -1.69 | -1.03 | -0.91 | -1.37 | -2.41 |
| 20 | -1.26 | -2.71 | -2.05 | -3.12 | -1.66 | -1.81 | -0.44 | -1.64 |
| 21 | -0.97 | -1.12 | -1.20 | -1.20 | -0.84 | -0.93 | -1.44 | -1.83 |
| 22 | -0.18 | -0.88 | -0.88 | -0.91 | -0.66 | -0.35 | 0.63 | 1.30 |
| 23 | -0.49 | -1.33 | -1.36 | -1.58 | -1.19 | -1.10 | -1.28 | -1.65 |
| 24 | -1.16 | -2.00 | -1.91 | -2.23 | -1.54 | -1.66 | -0.93 | -1.33 |
| 25 | -0.49 | -1.25 | -1.42 | -1.35 | -1.28 | -1.53 | -0.61 | -0.10 |
| 26 | -0.83 | -1.20 | -1.20 | -1.29 | -0.92 | -0.98 | -0.19 | -0.22 |
| 27 | -0.87 | -1.46 | -1.59 | -1.73 | -1.13 | -1.35 | -1.77 | -2.59 |
| 28 | -1.66 | -1.82 | -2.06 | -2.09 | -1.28 | -1.96 | -2.26 | -2.59 |

**Table D25. Yellowness Indexes measured in vials (YI-Vial) for samples stored at 25°C**

| Run | 0 weeks | 2 weeks | 4 weeks | 6 weeks | 8 weeks | 10 weeks | 12 weeks | 24 weeks |
|---|---|---|---|---|---|---|---|---|
| 1 | -1.12 | -0.28 | 0.15 | 0.28 | -1.88 | 0.98 | 1.33 | 2.71 |
| 2 | -1.51 | 2.37 | 3.83 | 4.27 | 0.57 | 4.84 | 5.54 | 6.20 |
| 3 | -1.49 | 0.71 | 1.65 | 2.70 | 4.71 | 3.48 | 3.98 | 4.81 |
| 4 | -0.19 | 3.59 | 4.92 | 4.93 | 3.32 | 5.33 | 5.48 | 6.42 |
| 5 | -0.89 | -0.15 | 0.37 | 0.99 | 5.32 | 1.79 | 2.20 | 3.56 |
| 6 | -1.55 | -0.81 | 0.28 | 1.64 | 1.29 | 3.43 | 5.01 | 11.74 |
| 7 | -0.88 | 2.31 | 4.72 | 7.42 | 2.46 | 11.79 | 15.14 | 25.89 |
| 8 | -1.54 | 0.58 | 2.22 | 4.77 | 11.08 | 10.19 | 12.47 | 24.93 |

(continued)

| Run | 0 weeks | 2 weeks | 4 weeks | 6 weeks | 8 weeks | 10 weeks | 12 weeks | 24 weeks |
|---|---|---|---|---|---|---|---|---|
| 9 | -0.37 | 0.05 | 0.51 | 0.60 | 7.78 | 1.07 | 1.36 | 2.06 |
| 10 | -0.94 | 0.85 | 2.49 | 4.12 | 1.05 | 8.25 | 9.45 | 20.29 |
| 11 | -0.82 | -0.61 | -0.25 | 0.07 | 6.01 | 1.22 | 1.58 | 3.52 |
| 12 | -0.82 | -0.01 | 0.28 | 0.55 | 0.65 | 1.1 | 1.42 | 3.67 |
| 13 | -0.78 | 0.84 | 0.51 | 1.49 | 0.94 | 1.84 | 2.18 | 4.32 |
| 14 | -0.97 | -0.33 | 0.72 | 1.88 | 1.75 | 3.32 | 3.89 | 4.12 |
| 15 | -1.77 | -0.96 | -0.57 | -0.17 | 2.35 | 0.21 | 0.38 | 1.28 |
| 16 | -0.58 | 4.15 | 5.71 | 9.46 | 0.06 | 13.45 | 17.23 | 39.4 |
| 17 | -1.44 | 0.57 | 1.60 | 2.43 | 11.99 | 4.48 | 5.60 | 11.15 |
| 18 | -0.94 | 0.69 | 2.16 | 3.75 | 3.28 | 6.63 | 8.38 | 16.60 |
| 19 | -0.72 | 0.87 | 0.41 | 0.70 | 5.01 | 1.96 | 1.95 | 1.71 |
| 20 | -1.26 | 3.56 | 6.16 | 9.92 | 2.05 | 16.87 | 20.61 | 35.28 |
| 21 | -0.97 | -0.12 | 0.18 | 0.33 | 13.99 | 0.75 | 1.20 | 1.96 |
| 22 | -0.18 | 3.62 | 6.58 | 9.38 | 0.56 | 14.28 | 17.38 | 27.05 |
| 23 | -0.49 | 0.58 | 0.89 | 1.25 | 12.17 | 1.47 | 3.00 | 2.44 |
| 24 | -1.16 | 1.41 | 3.47 | 5.37 | 1.13 | 8.45 | 10.34 | 18.11 |
| 25 | -0.49 | 1.40 | 3.45 | 6.16 | 7.08 | 8.92 | 11.07 | 21.07 |
| 26 | -0.83 | 1.35 | 3.86 | 6.15 | 6.95 | 10.71 | 13.13 | 22.11 |
| 27 | -0.87 | 0.45 | 0.70 | 2.62 | 8.04 | 3.94 | 4.78 | 9.24 |
| 28 | -1.66 | 0.05 | 1.09 | 3.07 | 3.72 | 4.23 | 6.65 | 10.20 |

**Table D30. Yellowness Indexes measured in vials (YI-Vial) for samples stored at 30°C**

| Run | 0 weeks | 2 weeks | 4 weeks | 6 weeks | 8 weeks | 10 weeks | 12 weeks | 24 weeks |
|---|---|---|---|---|---|---|---|---|
| 1 | -1.12 | 0.09 | 0.59 | 0.98 | 1.72 | 1.88 | 2.74 | 4.76 |
| 2 | -1.51 | 4.69 | 5.90 | 5.22 | 6.80 | 7.01 | 7.77 | 9.40 |
| 3 | -1.49 | 1.96 | 3.15 | 3.45 | 3.71 | 4.16 | 4.22 | 6.16 |
| 4 | -0.19 | 4.79 | 4.62 | 5.29 | 6.02 | 6.25 | 6.67 | 8.81 |
| 5 | -0.89 | 0.48 | 1.25 | 1.99 | 2.63 | 3.48 | 3.67 | 4.35 |
| 6 | -1.55 | 0.88 | 3.37 | 5.63 | 7.84 | 9.46 | 11.96 | 23.81 |
| 7 | -0.88 | 5.81 | 11.56 | 15.74 | 20.36 | 24.06 | 28.28 | 43.64 |
| 8 | -1.54 | 2.51 | 7.42 | 11.89 | 16.83 | 22.02 | 26.04 | 44.82 |
| 9 | -0.37 | 0.81 | 0.97 | 1.06 | 1.71 | 1.46 | 2.30 | 3.61 |
| 10 | -0.94 | 2.95 | 7.12 | 11.49 | 16.52 | 19.63 | 23.65 | 41.05 |
| 11 | -0.82 | -0.14 | 0.68 | 3.39 | 2.28 | 2.91 | 3.18 | 4.13 |
| 12 | -0.82 | 1.27 | 1.69 | 0.83 | 2.40 | 2.95 | 3.24 | 6.89 |
| 13 | -0.78 | 0.44 | 0.47 | 0.90 | 1.80 | 2.63 | 3.72 | 9.02 |
| 14 | -0.97 | 1.79 | 2.37 | 3.34 | 4.92 | 4.39 | 5.28 | 5.56 |
| 15 | -1.77 | -0.45 | 0.05 | 0.37 | 1.63 | 0.96 | 1.38 | 2.54 |
| 16 | -0.58 | 7.12 | 12.98 | 17.27 | 20.32 | 25.02 | 28.69 | 41.36 |

(continued)

| Run | 0 weeks | 2 weeks | 4 weeks | 6 weeks | 8 weeks | 10 weeks | 12 weeks | 24 weeks |
|---|---|---|---|---|---|---|---|---|
| 17 | -1.44 | 1.48 | 3.90 | 5.05 | 7.02 | 8.62 | 10.56 | 18.20 |
| 18 | -0.94 | 2.94 | 5.71 | 9.41 | 12.49 | 15.25 | 17.54 | 28.48 |
| 19 | -0.72 | 1.50 | 1.10 | 1.96 | 1.95 | 2.19 | 2.47 | 4.94 |
| 20 | -1.26 | 8.46 | 15.96 | 23.35 | 29.47 | 33.71 | 36.82 | 46.06 |
| 21 | -0.97 | 0.21 | 0.52 | 1.18 | 1.07 | 1.56 | 1.68 | 3.33 |
| 22 | -0.18 | 8.08 | 15.03 | 21.21 | 25.51 | 28.36 | 33.86 | 47.60 |
| 23 | -0.49 | 1.08 | 1.13 | 2.65 | 2.95 | 2.34 | 2.33 | 4.18 |
| 24 | -1.16 | 3.46 | 7.64 | 10.39 | 13.90 | 16.30 | 18.36 | 30.04 |
| 25 | -0.49 | 4.10 | 9.04 | 10.95 | 14.94 | 17.43 | 22.26 | 39.10 |
| 26 | -0.83 | 3.68 | 8.79 | 12.5 | 16.32 | 20.04 | 22.18 | 37.55 |
| 27 | -0.87 | 0.88 | 2.29 | 3.90 | 6.36 | 6.99 | 8.72 | 15.92 |
| 28 | -1.66 | 1.13 | 2.88 | 4.78 | 6.39 | 8.10 | 10.63 | 17.97 |

**Table D40. Yellowness Indexes measured in vials (YI-Vial) for samples stored at 40°C**

| Run | 0 weeks | 2 weeks | 4 weeks | 6 weeks | 8 weeks | 10 weeks | 12 weeks | 24 weeks |
|---|---|---|---|---|---|---|---|---|
| 1 | -1.12 | 1.43 | 3.13 | 4.73 | 6.11 | 6.77 | 8.39 | 14.04 |
| 2 | -1.51 | 6.24 | 8.50 | 10.16 | 12.23 | 12.67 | 13.36 | 19.28 |
| 3 | -1.49 | 3.71 | 5.12 | 5.83 | 7.22 | 8.25 | 8.74 | 12.69 |
| 4 | -0.19 | 6.90 | 8.48 | 9.72 | 10.87 | 11.99 | 12.57 | 17.65 |
| 5 | -0.89 | 2.25 | 3.62 | 4.15 | 4.98 | 5.27 | 5.7 | 8.63 |
| 6 | -1.55 | 9.43 | 15.76 | 21.7 | 26.34 | 29.73 | 35.86 | 47.29 |
| 7 | -0.88 | 28.17 | 40.08 | 48.21 | 53.75 | 60.46 | 61.18 | 69.48 |
| 8 | -1.54 | 22.18 | 31.82 | 41.67 | 50.52 | 56.46 | 63.32 | 66.58 |
| 9 | -0.37 | 1.82 | 3.00 | 4.17 | 5.45 | 6.10 | 6.94 | 11.56 |
| 10 | -0.94 | 16.58 | 28.98 | 36.78 | 44.83 | 53.55 | 56.97 | 64.83 |
| 11 | -0.82 | 1.87 | 3.32 | 4.12 | 4.73 | 5.51 | 5.89 | 8.51 |
| 12 | -0.82 | 0.89 | 2.69 | 4.72 | 6.91 | 9.58 | 11.72 | 25.05 |
| 13 | -0.78 | 0.85 | 3.25 | 5.95 | 9.06 | 11.75 | 14.84 | 27.07 |
| 14 | -0.97 | 3.90 | 4.66 | 5.10 | 5.61 | 6.26 | 6.74 | 10.10 |
| 15 | -1.77 | 0.41 | 1.61 | 2.15 | 3.11 | 3.96 | 4.48 | 8.24 |
| 16 | -0.58 | 25.18 | 34.98 | 42.11 | 48.36 | 54.10 | 55.03 | 60.20 |
| 17 | -1.44 | 6.54 | 11.93 | 16.54 | 20.03 | 23.66 | 26.77 | 41.25 |
| 18 | -0.94 | 14.02 | 21.89 | 26.30 | 32.09 | 35.65 | 39.77 | 44.78 |
| 19 | -0.72 | 2.61 | 3.25 | 4.51 | 5.69 | 6.14 | 7.36 | 11.44 |
| 20 | -1.26 | 31.06 | 42.74 | 44.55 | 45.70 | 46.06 | 45.98 | 47.55 |
| 21 | -0.97 | 1.02 | 2.01 | 3.00 | 4.16 | 5.13 | 5.37 | 9.24 |
| 22 | -0.18 | 29.45 | 38.76 | 46.82 | 52.94 | 60.04 | 64.65 | 75.87 |
| 23 | -0.49 | 1.61 | 2.73 | 3.62 | 4.52 | 5.46 | 5.62 | 9.52 |
| 24 | -1.16 | 17.49 | 28.06 | 32.15 | 34.62 | 32.25 | 33.25 | 35.41 |

(continued)

| Run | 0 weeks | 2 weeks | 4 weeks | 6 weeks | 8 weeks | 10 weeks | 12 weeks | 24 weeks |
|---|---|---|---|---|---|---|---|---|
| 25 | -0.49 | 16.10 | 25.87 | 34.57 | 42.69 | 50.83 | 58.25 | 74.75 |
| 26 | -0.83 | 15.79 | 26.40 | 33.59 | 39.99 | 47.21 | 52.12 | 69.61 |
| 27 | -0.87 | 3.70 | 8.63 | 11.68 | 15.45 | 19.02 | 21.10 | 32.21 |
| 28 | -1.66 | 6.38 | 12.64 | 16.93 | 20.78 | 25.36 | 28.42 | 45.31 |

The Yellowness Indexes YI(E313-96)(D65) measured by Vial method (YI-Vial) from initial time $t_0$ to 24 weeks (0, 2, 4, 6, 8 10,12, 24 weeks) from Tables DV5, DV25, DV30, and DV40 were plotted against time by storage temperature in FIG. 1. It was shown that YI-Vial maintained at very low levels for all the experimental runs at 5°C, indicating that the color change is negligible at 5°C storage temperature. However, as storage temperature was increased, the YI values increased, as did the variation between the runs or excipient factor combinations.

The inventors switched the Yellowness Indexes YI(E313-96)(D65) measurement method from Vial to Cuvette at 24 weeks and used the Cuvette method moving forward; hence, only 24-week YI-Cuvette data at different storage temperatures are analyzed and presented as the DoE responses in this disclosure. It is worth noting that YI-Vial and YI-Cuvette are highly correlated based on the 24-week data. The correlation coefficients between YI-Vial and YI-Cuvette at 24 weeks are provided in **Table 2** below.

**Table 2. Correlation Coefficients between YI-Vial and YI-Cuvette at 24 weeks at Different Storage Temperatures**

| Storage Temperature | 5°C | 25°C | 30°C | 40°C | Overall |
|---|---|---|---|---|---|
| Correlation Coefficient (*r*) | 0.8659 | 0.9764 | 0.9973 | 0.9989 | 0.9956 |

Yellowness Indexes YI(E313-96)(D65) were measured by cuvette method (YI-Cuvette) at 24 weeks and recorded in Table DC below.

**Table DC. Yellowness Indexes measured in cuvettes (YI-Cuvettes) for samples stored at 5°C, 25°C, 30°C, and 40°C for 24 weeks**

| Run | 5°C | 25°C | 30°C | 40°C |
|---|---|---|---|---|
| 1 | -7.48 | 0.16 | 2.36 | 9.35 |
| 2 | -6.02 | 4.11 | 7.46 | 16.19 |
| 3 | -7.6 | 2.37 | 3.74 | 8.30 |
| 4 | -4.81 | 3.69 | 6.89 | 12.75 |
| 5 | -8.00 | 1.49 | 2.49 | 4.58 |
| 6 | -6.83 | 10.58 | 21.4 | 46.13 |
| 7 | -4.31 | 25.14 | 43.65 | 67.41 |
| 8 | -6.58 | 24.93 | 48.90 | 66.04 |
| 9 | -7.00 | 0.35 | 2.55 | 7.51 |
| 10 | -6.23 | 17.36 | 39.88 | 64.8 |
| 11 | -7.50 | 0.80 | 2.50 | 5.68 |
| 12 | -6.66 | 2.35 | 4.78 | 22.5 |
| 13 | -7.21 | 2.14 | 8.28 | 23.32 |
| 14 | -7.75 | 2.50 | 3.90 | 5.97 |
| 15 | -8.85 | -1.19 | 0.16 | 4.48 |
| 16 | -3.34 | 25.48 | 43.95 | 56.77 |
| 17 | -6.48 | 8.96 | 16.75 | 39.30 |

(continued)

| Run | 5°C | 25°C | 30°C | 40°C |
|---|---|---|---|---|
| 18 | -5.76 | 15.53 | 27.96 | 39.74 |
| 19 | -6.51 | 0.16 | 2.25 | 7.49 |
| 20 | -4.41 | 34.20 | 43.80 | 43.93 |
| 21 | -5.62 | -0.46 | 1.55 | 4.93 |
| 22 | -2.93 | 23.98 | 46.25 | 74.69 |
| 23 | -6.67 | 0.46 | 2.11 | 5.31 |
| 24 | -6.19 | 14.43 | 31.03 | 30.85 |
| 25 | -5.11 | 18.33 | 37.84 | 69.91 |
| 26 | -3.86 | 21.47 | 36.34 | 67.15 |
| 27 | -6.72 | 6.44 | 13.52 | 30.22 |
| 28 | -6.69 | 7.77 | 16.68 | 41.97 |

The stepwise model selection technique was employed to produce candidate terms for each statistical model. To remain in the model, a term needed to be statistically significant at the commonly accepted 0.05 level of statistical significance (either one-sided or two-sided, as appropriate), unless the term was required to retain model hierarchy when a strong interaction effect was included in the model. In addition, all terms in the regression models needed to increase $R^2$ for prediction to a statistically meaningful degree.

**Table A1** summarizes the statistically significant factor effects on each response. All model coefficients listed in **Table A1** were fitted using the "-1" and "+1" coded levels instead of actual levels for each factor. The actual levels for each coded factor were provided in **Table A2.**

**Table A1: Summary of Factor Effects and Fitted Models for Each of the Four Responses**

| **Response** | | **YI-Cuvette at 24 weeks at 5°C** | **YI-Cuvette at 24 weeks at 25°C** | **YI-Cuvette at 24 weeks at 30°C** | **YI-Cuvette at 24 weeks at 40°C** |
|---|---|---|---|---|---|
| **Data Summary** | **Mean** | -6.18 | 9.77 | 18.53 | 31.33 |
| | **Standard Deviation** | 1.44 | 10.32 | 17.44 | 24.74 |
| | **Minimum** | -8.85 | -1.19 | 0.16 | 4.48 |
| | **Maximum** | -2.93 | 34.2 | 48.9 | 74.69 |

(continued)

| Response | | YI-Cuvette at 24 weeks at 5°C | YI-Cuvette at 24 weeks at 25°C | YI-Cuvette at 24 weeks at 30°C | YI-Cuvette at 24 weeks at 40°C |
|---|---|---|---|---|---|
| Model Coefficients (*p-value*) | Transformation | Natural Log: ln(Y + 9.73) | Square Root: Sqrt(Y + 1.31) | Square Root: Sqrt(Y) | Natural Log: ln(Y) |
| | Intercept | 1.5442 | 4.8527 | 5.7805 | 3.7685 |
| | A: Added BHT | 0.2452 (< *0.0001*) | 1.0963 (< *0.0001*) | 1.6058 (< *0.0001*) | 0.9170 (< *0.0001*) |
| | B: Added Tocopherol | 0.2107 (< 0.0001) | 0.6810 (< *0.0001)* | 0.9768 (< *0.0001)* | 0.3162 (< *0.0001)* |
| | C: White Petrolatum | | -0.2174 (*0.0180*) | -0.1493 *(0.0119)* | |
| | D: Oleyl Alcohol | 0.0825 *(0.0489)* | 0.1000 *(0.2664)* | 0.1230 *(0.0388)* | |
| | E: PEG 400 | 0.0652 *(0.1036)* | 0.2583 *(0.0063)* | 0.2887 (< *0.0001)* | 0.1295 (*0.0080*) |
| | AB | | 0.5044 (< *0.0001*) | 0.6440 (< *0.0001*) | |
| | AC | | | 0.3115 (*0.0001*) | |
| | AD | -0.1926 (*0.0002*) | | | |
| | AE | -0.2151 (< *0.0001*) | | | |
| | BE | | | -0.1962 (*0.0045*) | |
| | CD | | -0.1757 (*0.0538*) | -0.1512 (*0.0115*) | |
| | $A^2$ | | -1.4044 (< *0.0001)* | -1.9667 (< *0.0001)* | -0.8650 (< *0.0001)* |
| | $B^2$ | -0.4108 *(0.0018)* | -0.9116 (0.0023) | -0.5233 *(0.0056)* | |
| Fit summary | Adjusted $R^2$ | 0.7879 | 0.9264 | 0.9845 | 0.9469 |
| | Predicted $R^2$ | 0.7044 | 0.8794 | 0.9728 | 0.9275 |
| | RMSE | 0.2019 | 0.4350 | 0.2701 | 0.2346 |

**Table A2: Summary of Factor Coding for Statistical Models Presented in Tables A1**

| Factor | Unit | Type | Low Level | High Level | Coded | Values |
|---|---|---|---|---|---|---|
| A: Added BHT | ppm | Numeric | 0 | 30 | -1=0 | +1=30 |
| B: Added Tocopherol | ppm | Numeric | 0 | 30 | -1=0 | +1=30 |
| C: White Petrolatum | NA | Categoric | Calumet | Croda | -1=Calumet | +1=Croda |
| D: Oleyl Alcohol | NA | Categoric | BASF | Croda | -1=BASF | +1=Croda |
| E: PEG 400 | NA | Categoric | Dow | Croda | -1=Dow | +1=Croda |

The list below explains three of the important statistics that have been included in Table A1.

1) The adjusted $R^2$ value expresses the percentage of total variation in the data that has been explained by the model, after adjusting for the number of terms in the model compared to the number of runs in the experiment. The closer the adjusted $R^2$ is to 1.00, the better the model fits the data observed in this experiment.
2) The $R^2$ for prediction ("predicted $R^2$") estimates the percentage of total variation in new data that would be explained by a model with the same terms as the model derived in this experiment. The closer the $R^2$ for prediction is to 1.00, the

better the predictive power of the model.

3) The root mean square error (RMSE) is calculated from the individual differences between each experimental run's observed and predicted values. This difference is called a "residual". The RMSE can be interpreted as the standard deviation of the residuals.

The models in **Table A1** are also presented using "actual" factor values in **Table A3.** The excipient levels for each excipient combination ID can be found in **Table A4.**

**Table A3: Final Equations in Terms of Actual Factors for the Models in Table A1**

| Response | Excipient Combinati on ID* | Final Equation in Terms of Actual Factors | |
|---|---|---|---|
| **YI-Cuvette at 24 weeks at 5°C** | **1, 5** | $\exp(0.122155 + 0.043522\times A + 0.068824\times B - 0.001826\times B^2) - 9.73$ | |
| | **2, 6** | $\exp(0.682757 + 0.014842\times A + 0.068824\times B - 0.001826\times B^2) - 9.73$ | |
| | **3, 7** | $\exp(0.672228 + 0.017847\times A + 0.068824\times B - 0.001826\times B^2) - 9.73$ | |
| | **4, 8** | $\exp(1.232829 - 0.010833\times A + 0.068824\times B - 0.001826\times B^2) - 9.73$ | |
| **YI-Cuvette at 24 weeks at 25°C** | **1** | Constant = 0.947113 | $(\text{Constant} + 0.226715\times A + 0.133321\times B + 0.002242\times A\times B - 0.006242\times A^2 - 0.004052\times B^2)^2 - 1.31$ |
| | **2** | Constant = 1.463627 | |
| | **3** | Constant = 1.498661 | |
| | **4** | Constant = 2.015175 | |
| | **5** | Constant = 0.863770 | |
| | **6** | Constant = 1.380284 | |
| | **7** | Constant = 0.712325 | |
| | **8** | Constant = 1.228840 | |
| **YI-Cuvette at 24 weeks at 30°C** | **1** | Constant = 1.053719 | $(\text{Constant} + 0.305578\times A + 0.105034\times B + 0.002862\times A\times B - 0.008741\times A^2 - 0.002326\times B^2)^2$ |
| | **3** | Constant = 1.601948 | |
| | **2** | Constant = 2.023545 | $(\text{Constant} + 0.305578\times A + 0.078872\times B + 0.002862\times A\times B - 0.008741\times A^2 - 0.002326\times B^2)^2$ |
| | **4** | Constant = 2.571774 | |
| | **5** | Constant = 0.434579 | $(\text{Constant} + 0.347109\times A + 0.105034\times B + 0.002862\times A\times B - 0.008741\times A^2 - 0.002326\times B^2)^2$ |
| | **7** | Constant = 0.378150 | |
| | **6** | Constant = 1.404405 | $(\text{Constant} + 0.347109\times A + 0.078872\times B + 0.002862\times A\times B - 0.008741\times A^2 - 0.002326\times B^2)^2$ |
| | **8** | Constant = 1.347976 | |
| **YI-Cuvette at 24 weeks at 40°C** | **1, 3, 5, 7** | Constant = 1.540698 | $\exp(\text{Constant} + 0.176473\times A + 0.021081\times B - 0.003845\times A^2)$ |
| | **2, 4, 6, 8** | Constant = 1.799785 | |

* The excipient levels for each excipient combination ID can be found in **Table A4.**

**Table A4. Excipient Combinations**

| Excipient Combination ID | White Petrolatum | Oleyl Alcohol | PEG 400 |
|---|---|---|---|
| 1 | Calumet™ | BASF™ | Dow™ |
| 2 | Calumet™ | BASF™ | Croda™ |
| 3 | Calumet™ | Croda™ | Dow™ |
| 4 | Calumet™ | Croda™ | Croda™ |
| 5 | Croda™ | BASF™ | Dow™ |
| 6 | Croda™ | BASF™ | Croda™ |

(continued)

| Excipient Combination ID | White Petrolatum | Oleyl Alcohol | PEG 400 |
|---|---|---|---|
| 7 | Croda™ | Croda™ | Dow™ |
| 8 | Croda™ | Croda™ | Croda™ |

Using the information presented in **Tables A1-A4,** one could predict the mean values of 24-week YI-Cuvette at different storage temperatures for the excipient combination of interest. For example, to predict the mean value of 24-week YI-Cuvette at 40°C for excipient combination #1 (Calumet White Petrolatum, BASF Oleyl Alcohol and Dow PEG 400) with 0 ppm Added BHT and 0 ppm Added Tocopherol, one could use the coded values of the factors (A = -1, B = -1, C = -1, D = -1, E = -1) and the model coefficients from **Table A1** or use the actual values of the factors (A = 0 ppm, B = 0 ppm) and the equation from **Table A3.**

For example, using the equation of 24-week YI-Cuvette at 40°C for excipient combination #1 in Table A3:

$$\exp(1.540698 + 0.176473\times A + 0.021081\times B - 0.003845\times A^2) = YI\ \#1$$

and plugging in the actual values of A = 0 ppm and B = 0 ppm, one can calculate

$$\exp(1.540698 + 0.176473\times 0 + 0.021081\times 0 - 0.003845\times 0^2) = 4.67;$$

which is the predicted median and is different from the predicted mean on the original scale. In this case, there is a correction factor $SD^2/2$ where SD is the standard deviation of the prediction on the transformed scale. Thus, the predicted mean of 24-week YI-Cuvette at 40°C for excipient combination #1 on the original scale can be calculated as:

$$\exp(1.540698 + 0.176473\times 0 + 0.021081\times 0 - 0.003845\times 0^2 + SD^2/2) = \exp(1.540698+0.2346^2/2) \approx 4.80.$$

It is worth noting that when a predicted mean response on a transformed scale is converted back to its original scale, the predicted mean becomes the predicted median. Design-Expert® applies a correction automatically to the back-transformed prediction, so it correctly predicts the mean, rather than the median. The outputs in **Tables 0, 3, 4** are the corrected predicted means.

Detailed information about each model has been provided below, including contour plots in the figures that allow the magnitude and practical importance of the effects to be evaluated. Added BHT and/or Added Tocopherol affected all four responses. These contour plots consist of "contour lines" (lines of constant predicted response values) with Added Tocopherol and Added BHT as X and Y axes, respectively.

**YI-Cuvette at 24 weeks at 5°C**

As best seen in Table DC above, at 5°C, 24-week YI-Cuvette values were below 0 for all 28 runs and ranged from -8.85 for run 15 to -2.93 for run 22. Added BHT, Added Tocopherol, Oleyl Alcohol, and PEG 400 affected YI-Cuvette at 24 weeks at 5°C. There was a statistically significant quadratic effect of Added Tocopherol and two-way interaction effects of Added BHT and Oleyl Alcohol, and Added BHT and PEG 400.

With the fitted DoE models, the predicted means of YI-Cuvette at 24 weeks at 5°C could be used to select the best-case and worst-case color-minimizing excipient compositions. The contour plots of the theoretical best and the worst-case scenarios in terms of the lowest and the highest predicted YI-Cuvette at 24 weeks at 5°C with 0 ppm Added BHT and 0 ppm Added Tocopherol are provided in FIGS. 2 and 3, respectively. Under these conditions, the theoretical best-case scenario illustrated in FIG. 2 was predicted for samples including Calumet™ White Petrolatum, BASF™ Oleyl Alcohol and Dow™ PEG 400. The worst-case scenario illustrated in FIG. 3 was predicted for samples including Calumet™ White Petrolatum, Croda™ Oleyl Alcohol and Croda™ PEG 400.

**YI-Cuvette at 24 weeks at 25°C**

As best seen in Table DC above, at 25°C, 24-week YI-Cuvette ranged from -1.19 for run 15 to 34.2 for run 20 across the 28 runs. All five factors affected YI-Cuvette at 24 weeks at 25°C. Added BHT and Added Tocopherol had statistically significant quadratic and two-way interaction effects on YI-Cuvette at 24 weeks. There was also a statistically significant main effect of PEG 400 and a weakly statistically significant ($p$ = 0.0538) two-way interaction effect of White

Petrolatum and Oleyl Alcohol.

With the fitted DoE models, the predicted means of YI-Cuvette at 24 weeks at 25°C could be used to select the best-case and worst-case color-minimizing excipient compositions. The contour plots of the theoretical best and the worst-case scenarios in terms of the lowest and the highest predicted YI-Cuvette at 24 weeks at 25°C with 0 ppm Added BHT and 0 ppm Added Tocopherol are provided in FIGS. 4 and 5, respectively. Under these conditions, the best-case scenario illustrated in FIG. 4 was predicted for samples including Croda™ White Petrolatum, Croda™ Oleyl Alcohol and Dow™ PEG 400. The worst-case scenario illustrated in FIG. 5 was predicted for samples including Calumet White Petrolatum, Croda™ Oleyl Alcohol and Croda™ PEG 400. By comparing across the rows of Table 0, the effect of varying storage temperature can be understood. The effects of storage temperature are also seen by comparing the predicted means and the contour plots of FIGS. 2 and 3 to FIGS. 4 and 5.

**YI-Cuvette at 24 weeks at 30°C**

As best seen in Table DC above, at 30°C, 24-week YI-Cuvette ranged from 0.16 for run 15 to 48.9 for run 8 across the 28 runs. All five factors affected YI-Cuvette at 24 weeks at 30°C. Added BHT and Added Tocopherol had statistically significant quadratic and two-way interaction effects on YI-Cuvette at 24 weeks. There were also three statistically significant two-way interaction effects: Added BHT and White Petrolatum, Added Tocopherol and PEG 400, and White Petrolatum and Oleyl Alcohol.

With the fitted DoE models, the predicted means of YI-Cuvette at 24 weeks at 30°C could be used to select the best-case and worst-case color-minimizing excipient compositions. The contour plots of the best and the worst-case scenarios in terms of the lowest and the highest predicted YI-Cuvette at 24 weeks at 30°C with 0 ppm Added BHT and 0 ppm Added Tocopherol are provided in FIGS. 6 and 7, respectively. Under these conditions, the best-case scenario illustrated in FIG. 6 was predicted for samples including Croda™ White Petrolatum, Croda™ Oleyl Alcohol and Dow™ PEG 400. The worst-case scenario illustrated in FIG. 7 was predicted for samples including Calumet White Petrolatum, Croda™ Oleyl Alcohol and Croda™ PEG 400. By comparing across the rows of Table 0, the effect of varying storage temperature can be understood. The effects of storage temperature are also seen by comparing the predicted means and the contour plots of earlier figures to FIGS. 6 and 7.

**YI-Cuvette at 24 weeks at 40°C**

As best seen in Table DC above, at 40°C, 24-week YI-Cuvette ranged from 4.48 for run 15 to 74.69 for run 22 across the 28 runs. Added BHT, Added Tocopherol, and PEG 400 affected YI-Cuvette at 24 weeks at 40°C. There was a statistically significant quadratic effect of Added BHT, and main effects of Added Tocopherol and PEG 400.

With the fitted DoE models, the predicted means of YI-Cuvette at 24 weeks at 40°C could be used to select the best-case and worst-case color-minimizing excipient compositions. The contour plots of the best and the worst-case scenarios in terms of the lowest and the highest predicted YI-Cuvette at 24 weeks at 40°C with 0 ppm Added BHT and 0 ppm Added Tocopherol are provided in FIGS. 8 and 9, respectively. FIG 8A shows an enlarged or zoomed-in version of FIG. 8 with 0 to 2 ppm of Added BHT and 0 to 2 ppm of Added Tocopherol. Under these conditions, the best-case scenario illustrated in FIG. 8 was predicted for samples including Calumet White Petrolatum, BASF™ Oleyl Alcohol and Dow™ PEG 400. FIG. 8A is an enlarged or zoomed-in contour plot of Yellowness Index (YI-Cuvette) taken from the lower left region of FIG. 8, and shows that low Yellowness area in greater detail. The worst-case scenario illustrated in FIG. 9 was predicted for samples including Calumet White Petrolatum, Croda™ Oleyl Alcohol and Croda™ PEG 400. By comparing across the rows of Table 0, the effect of varying storage temperature can be understood. The effects of storage temperature are also seen by comparing the predicted means and the contour plots of earlier figures to FIGS. 8 and 9.

**Statistical Conclusions**

Calumet™ White Petrolatum and mineral oil contain BHT and Tocopherol respectively in amounts that vary from lot-to-lot. White Petrolatum and mineral oil comprise 10% w/w and 5% w/w of the cream, respectively. Calumet™ White Petrolatum is stabilized with up to 20 ppm BHT and the lot used to prepare the DoE cream samples contained 5 ppm BHT, which corresponds to 0.5 ppm BHT in the cream. As such, 1.5 ppm Added BHT represents the worst-case scenario of a White Petrolatum lot that contains 20 ppm BHT. Calumet™ mineral oil is stabilized with up to 20 ppm tocopherol and the amount in the lot used to prepare the DoE samples was not quantified, therefore 1 ppm Added tocopherol is used to represent the worst-case scenario of a mineral oil lot that contains 20 ppm tocopherol. Based on the fitted models **(Table A3),** the predicted means (and the upper limits of their 95% confidence intervals) of YI-Cuvette at 24 weeks at different storage temperatures for all eight excipient combinations are provided in **Table 0.** Note that Added BHT was set to be 0 or 1.5 ppm and Added Tocopherol values was set to be 0 ppm for the calculations in **Table 0.** In addition, the predicted means (and the upper limits of their 95% confidence intervals) of YI-Cuvette at 24 weeks at different storage temperatures for the

eight excipient combinations with 0 or 1.5 ppm Added BHT and 1 ppm Added Tocopherol are provided in **Table 3** below.

**Table 3. The Predicted Means of YI-Cuvette at 24 Weeks with 1 ppm Added Tocopherol**

| Excipient Comb. ID | White Petrolatum | Oleyl Alcohol | PEG 400 | Added BHT (ppm) | Predicted Mean (Upper Limit of 95% Confidence Interval) of YI-Cuvette at 24 Weeks | | | |
|---|---|---|---|---|---|---|---|---|
| | | | | | 5°C | 25°C | 30°C | 40°C |
| 1 | Calumet™ | BASF™ | Dow™ | 0 | -8.50 (-8.18) | 0.04 (1.37) | 1.41 (2.29) | 4.90 (5.92) |
| | | | | 1.5 | -8.42 (-8.09) | 0.86 (2.38) | 2.63 (3.72) | 6.33 (7.54) |
| 2 | Calumet™ | BASF™ | Croda™ | 0 | -7.58 (-7.05) | 1.42 (3.33) | 4.48 (6.24) | 6.35 (7.66) |
| | | | | 1.5 | -7.53 (-7.92) | 2.58 (4.69) | 6.54 (8.51) | 8.20 (9.80) |
| 3 | Calumet™ | Croda™ | Dow™ | 0 | -7.60 (-7.13) | 1.53 (3.29) | 2.98 (4.21) | 4.90 (5.92) |
| | | | | 1.5 | -7.54 (-7.08) | 2.71 (4.69) | 4.69 (6.16) | 6.33 (7.54) |
| 4 | Calumet™ | Croda™ | Croda™ | 0 | -5.99 (-5.07) | 3.48 (5.84) | 7.09 (9.13) | 6.35 (7.66) |
| | | | | 1.5 | -6.05 (-5.19) | 5.00 (7.60) | 9.63 (11.90) | 8.20 (9.80) |
| 5 | Croda™ | BASF™ | Dow™ | 0 | -8.50 (-8.18) | -0.13 (1.19) | 0.36 (0.94) | 4.90 (5.92) |
| 6 | Croda™ | BASF™ | Croda™ | 0 | -7.58 (-7.05) | 1.16 (2.97) | 2.27 (3.48) | 6.35 (7.66) |
| 7 | Croda™ | Croda™ | Dow™ | 0 | -7.60 (-7.13) | -0.41 (0.67) | 0.30 (0.80) | 4.90 (5.92) |
| 8 | Croda™ | Croda™ | Croda™ | 0 | -5.99 (-5.07) | 0.72 (2.28) | 2.10 (3.18) | 6.35 (7.66) |

In one embodiment where the API concentration was between about 1% and about 3% by wt., Calumet White Petrolatum containing not more than 7 ppm BHT and Croda™ Oleyl Alcohol (Super Refined™ Novol™) were found to be suitable alternatives to existing excipient ingredients Croda™ Petrolatum (Crolatum™ V-SO) and BASF™ Oleyl Alcohol (Kollicream™ OA), respectively, as the changes do not impact product quality. This assessment is based on the results of a 24-week DoE that explored the impact of several factors, including petrolatum source (Calumet™ and Croda™), oleyl alcohol source (BASF™ and Croda™), and total (inherent and added) BHT, on drug product appearance (color) and purity as a function of storage time and temperature.

BHT is brought into the topical cream as an additive to an excipient, white petrolatum, which constitutes 10% w/w of the total cream composition. All clinical trial drug product lots manufactured to date were made with Calumet™ Super White™ Petrolatum USP, which is stabilized with up to 20 ppm BHT and available from Calumet Refining, Indianapolis, IN, USA. In the excipient DoE, the inventors explored a BHT-free petrolatum, Croda™ Crolatum™ V-SO, which is stabilized with 10 ppm tocopherol and available from Croda International, Snaith, East Riding of Yorkshire, UK.

Based on the results of the statistical analysis and additional manufacturing efficiency considerations, Combination 5 in **Table 4,** which uses Crolatum™ and Kollicream™ OA, was identified as the preferred combination to minimize yellow color development. Combinations 1 and 7, are predicted to also result in acceptable color development, when the total cream formulation contains not more than about 0.2 ppm added BHT **(FIG. 8A).** The Calumet White Petrolatum used in these samples contained about 5 ppm BHT, which at 10% petrolatum translates into 0.5 ppm BHT and when combined with the 0.2 ppm added BHT provides not more than about 0.7 ppm BHT total in the cream. Additionally, no degradants were observed in the DoE samples stored at 40°C for 24 weeks, which indicates a variety of excipient combinations are acceptable for product purity so long as the BHT limitations are maintained.

The experiments showed some limits on the amount of tocopherol in the formulation might also be beneficial

independently or in combination with the BHT limits. Fig. 8A, shows that if there is no added BHT in the formulation, acceptable color development can be achieved by limiting added tocopherol to less than about 2 ppm. Since other excipients in the formulation may inherently include tocopherol, such as the mineral oil, oleyl alcohol, and the white petrolatum, an overall limit of tocopherol in the total formulation of less than about 6.5 ppm is believed to lead to acceptable color development. In the case where there is less than about 0.7 ppm BHT present overall in the formulation, the tocopherol present overall in the formulation should be limited to about 5 ppm.

**Table 4. The Predicted Means of YI-Cuvette at 24 Weeks at Different Storage Temperatures for Combinations with 1 ppm Added Tocopherol-Acceptable Color Stability Combinations**

| Excipient Comb. ID | White Petrolatum | Oleyl Alcohol | PEG 400 | Predicted Mean (Upper Limit of 95% Confidence Interval) of YI-Cuvette at 24 Weeks | | | |
|---|---|---|---|---|---|---|---|
| | | | | 5°C | 25°C | 30°C | 40°C |
| 5 | Croda™ | BASF™ | Dow™ | -8.50 (-8.18) | -0.13 (1.19) | 0.36 (0.94) | 4.90 (5.92) |
| 1 | Calumet™ | BASF™ | Dow™ | -8.50 (-8.18) | 0.04 (1.37) | 1.41 (2.29) | 4.90 (5.92) |
| 7 | Croda™ | Croda™ | Dow™ | -7.60 (-7.13) | -0.41 (0.67) | 0.30 (0.80) | 4.90 (5.92) |

Formulations 5, 1, and 7 were prepared at API concentrations of 1% w/w and 3% and stored in foil laminate tubes for up to 16 weeks at 40 degrees C. Formulation 4 was also prepared, packaged, and stored under the same conditions to serve as a negative control. Yellowness Index or YI(E313-96)(D65) measurements were performed at 0, 4, and 16 weeks and are recorded in Table T1. The measurements were performed using disposable plastic cuvettes by sampling the creams through the orifice of the tubes. Thus, the measurements were Y-Cuvette measurements. The data for the 1% API concentration formulations is graphically presented in Fig. 10; and the data for the 3% API concentration formulations is plotted in Fig. 11.

**Table T. Yellowness Indexes measured in cuvettes for excipient combinations 5, 1, 7 and 4 stored in foil laminate tubes at 40°C for up to 16 weeks**

| API Concentration | 1% w/w | | | 3% w/w | | |
|---|---|---|---|---|---|---|
| Excipient Comb. ID | 0 weeks | 4 weeks | 16 weeks | 0 weeks | 4 weeks | 16 weeks |
| 5 | -2.80 | -1.53 | 1.63 | -1.71 | -0.46 | 3.50 |
| 1 | -2.09 | -0.36 | 4.01 | -1.72 | 1.86 | 7.69 |
| 7 | -2.74 | -1.32 | 2.74 | -1.22 | 0.48 | 5.94 |
| 4 | -3.09 | 1.28 | 6.05 | -2.22 | 3.93 | 10.58 |

As can be seen from the data, the formulations 1, 5, and 7 all maintained whiteness or relatively low YI as compared to the negative control formulation 4. At 3% API concentration, formulations 5 and 7, which were free of BHT, maintained a Yellowness Index of less than about 6 when stored for 16 weeks at 40°C. The above experiment detailed in Table T is on-going and it is projected that the Yellowness Index for formulations 5 and 7 will be less than about 10 at 24 weeks at 40°C. At 1% API concentration, the BHT-free formulations 5 and 7 maintained a Yellowness Index of less than about 3 when stored for 16 weeks at 40°C. It is projected that the Yellowness Index for formulations 5 and 7 will be less than about 6 at 24 weeks at 40°C.

The chemical structure of a first JAK inhibitor for which the formulation of this invention is useful is shown below. By way of example and not limitation, this JAK inhibitor is a JAK1, and more particularly is a TYK2/JAK1 inhibitor.

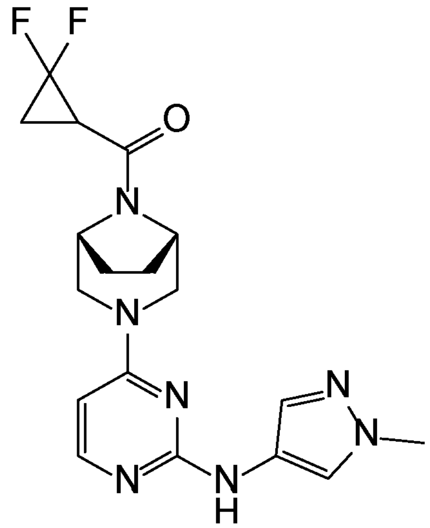

The chemical structure of a second JAK inhibitor for which the formulation of this invention is useful is shown below. By way of example and not limitation, this second JAK inhibitor is a TYK2/JAK1 inhibitor that is described in the co-owned application USSN 63/047606, filed Jul 2, 2020.

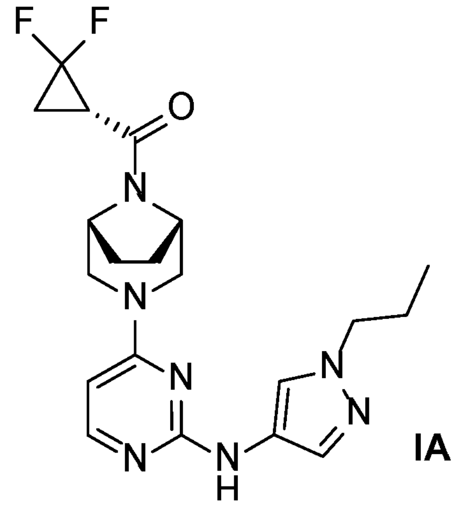

**IA**

The chemical name of this second JAK or JAK1/TYK2 inhibitor is:
((1R,5S)-3-(2-((1-propryl-1H-pyrazol-4-yl)amino)pyrimidin-4-yl)-3,8-diazabicyclo[3.2.1]octan-8-yl)((R)-spiro[2.3]hexan-1-yl)methanone.

Without being bound by the accuracy of this theory or hypothesis, it is believed that the BHT-related yellowing phenomena occurs via reaction of the API or structurally similar species with BHT to form a conjugated species with yellow color. In the proposed mechanism shown below with respect to the first JAK inhibitor as an example, BHT is oxidized through reaction with air, excipients, and/or excipient degradants. The oxidized BHT species can then react with the API or structurally similar species to form a more highly conjugated, yellow-colored species as shown in the following scheme. Based on the nature of the reaction, it is reasonable to infer that APIs that share the structural elements shown above would also be susceptible to this degradation mechanism in the presence of BHT and therefore would benefit from limiting the amount of BHT in view of the present invention.

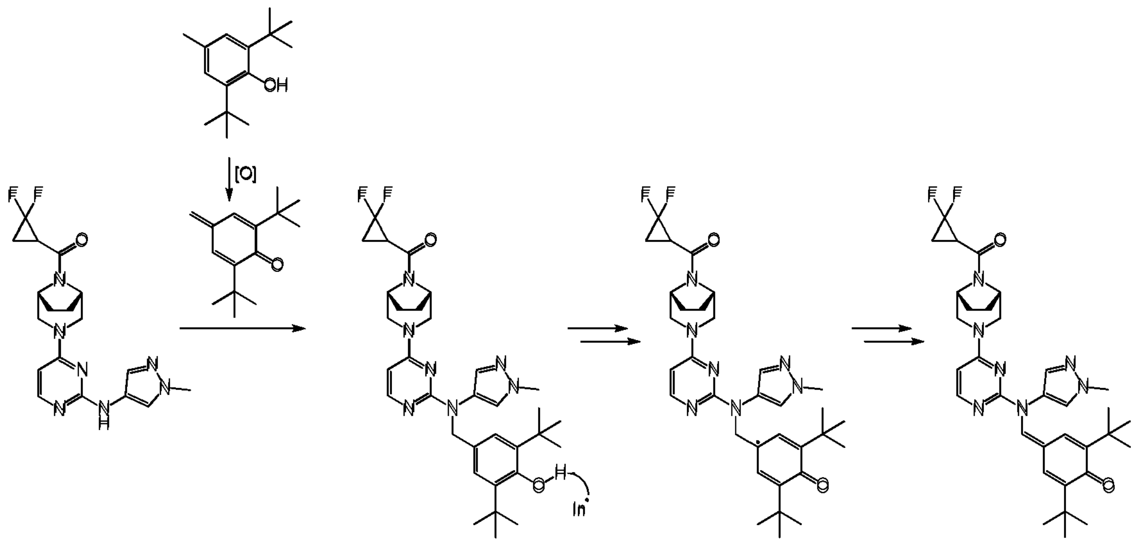


Based upon the foregoing it should be apparent that the present invention at least satisfies its stated objectives. A stable, low cost, easy to manufacture formulation is disclosed.

The present invention has been described with reference to specific details of embodiments thereof. It is not intended that such details be regarded as limitations upon the scope of the invention except insofar as and to the extent that they are included in the accompanying claims. For example, the formulation is not limited to the specific exemplary embodiments with ingredients or excipients from the specific suppliers identified. The formulation may include as substitutes an equivalent grade of ingredient or excipient from a different supplier and it is believed that the results would be similar in terms of color stability as long as the amount of BHT is limited as claimed.

## Claims

1. A topical formulation comprising:

   a JAK inhibitor in an oil-in-water emulsion;
   white petrolatum in an amount of 10% by wt.;
   less than 0.7 ppm butyl hydroxytoluene (BHT) by wt.;
   oleyl alcohol in an amount of 2% by wt.; and
   an antimicrobial agent.

2. The topical formulation of claim 1, wherein the JAK inhibitor is a JAK1 inhibitor.

3. The topical formulation of claim 1, wherein the JAK inhibitor is a TYK2/JAK1 inhibitor.

4. The topical formulation of claim 1, wherein the JAK inhibitor comprises ((S)-2,2-difluorocyclopropyl)-((1R,5S)-3-(2-((1-methyl-1H-pyrazol-4-yl)amino)pyrimidin-4-yl)-3,8-diazabicyclo[3.2.1]-octan-8-yl)methanone.

5. The topical formulation according to any one of claims 1 to 4, wherein the JAK inhibitor is present in an amount of 1% to 3% by wt.

6. The topical formulation according to any one of claims 1 to 5, comprising tocopherol in an amount of less than 6.5 ppm.

7. The topical formulation according to any one of claims 1 to 6, comprising polyethylene glycol 400 in an amount of 10% by wt.

8. The topical formulation according to any one of claims 1 to 7, comprising diethylene glycol monoethyl ether in an amount of 15% by wt. and optionally, emulsifying wax in an amount of 10% by wt.

9. The topical formulation according to any one of claims 1 to 8, wherein the antimicrobial agent comprises 2-phenoxyethanol in an amount of 1% by wt.

**10.** The topical formulation according to any one of claims 1 to 9, wherein the topical formulation maintains a white color having a yellowness index (YI) value of less than 10, measured by American Society for Testing and Materials (ASTM) Method E313, YI(E313-96)(D65), when stored for 24 weeks at 40 degrees C.

**11.** The topical formulation according to any one of claims 1 to 10, wherein the topical formulation maintains a white color having a yellowness index value of less than 6, measured by American Society for Testing and Materials (ASTM) Method E313, YI(E313-96)(D65), when stored for 16 weeks at 40 degrees C.

**12.** The topical formulation according to any one of claims 1 to 10, wherein the topical formulation has a yellowness index value that increases by not more than 8, measured by American Society for Testing and Materials (ASTM) Method E313, YI(E313-96)(D65), over 24 weeks at 40 degrees C.

**13.** The topical formulation according to any one of claims 1 to 12, wherein the white petrolatum includes not more than 7 ppm of BHT.

**14.** A topical formulation according to claim 1, further comprising:

tocopherol in an amount of less than 6.5 ppm;
polyethylene glycol 400 in an amount of 10% by wt.;
diethylene glycol monoethyl ether in an amount of 15% by wt.;
mineral oil in an amount of 5% by wt.;
an emulsifying wax in an amount of 10% by wt.; and
an antimicrobial agent comprises 2-phenoxyethanol in an amount of 1% by wt.;
wherein the topical formulation is free of BHT and thereby maintains a yellowness index of less than 6 when stored at 40 degrees C for 16 weeks and not more than 10 when stored for 24 weeks at 40 degrees C, measured by American Society for Testing and Materials (ASTM) Method E313, YI(E313-96)(D65).

**15.** The topical formulation according to any one of claims 1 to 14 for use in treating a dermatological condition is selected from the group consisting of psoriasis, atopic dermatitis, atopic eczema, chronic hand eczema, uticaria, vitiligo, cutaneous lupus and alopecia areata.

**Patentansprüche**

**1.** Topische Formulierung, umfassend:

einen JAK-Inhibitor in einer Öl-in-Wasser-Emulsion;
weiße Vaseline in einer Menge von 10 Gew.-%;
weniger als 0,7 ppm Butylhydroxytoluol (BHT) in Gew.-%;
Oleylalkohol in einer Menge von 2 Gew.-%; und
ein antimikrobielles Mittel.

**2.** Topische Formulierung nach Anspruch 1, wobei der JAK-Inhibitor ein JAK1-Inhibitor ist.

**3.** Topische Formulierung nach Anspruch 1, wobei der JAK-Inhibitor ein TYK2-/JAK1-Inhibitor ist.

**4.** Topische Formulierung nach Anspruch 1, wobei der JAK-Inhibitor ((S)-2,2-Difluorcyclopropyl)-((1R,5S)-3-(2-((1-Methyl-1H-pyrazol-4-yl)amino)pyrimidin-4-yl)-3,8-diazabicyclo[3.2.1]-octan-8-yl)methanon umfasst.

**5.** Topische Formulierung nach einem der Ansprüche 1 bis 4, wobei der JAK-Inhibitor in einer Menge von 1 bis 3 Gew.-% vorhanden ist.

**6.** Topische Formulierung nach einem der Ansprüche 1 bis 5, die Tocopherol in einer Menge von weniger als 6,5 ppm umfasst.

**7.** Topische Formulierung nach einem der Ansprüche 1 bis 6, umfassend Polyethylenglykol 400 in einer Menge von 10 Gew.-%.

**8.** Topische Formulierung nach einem der Ansprüche 1 bis 7, umfassend Diethylenglykolmonoethylether in einer Menge von 15 Gew.-% und gegebenenfalls Emulgierwachs in einer Menge von 10 Gew.-%.

**9.** Topische Formulierung nach einem der Ansprüche 1 bis 8, wobei das antimikrobielle Mittel 2-Phenoxyethanol in einer Menge von 1 Gew.-% umfasst.

**10.** Topische Formulierung nach einem der Ansprüche 1 bis 9, wobei die topische Formulierung eine weiße Farbe mit einem Vergilbungsindex (YI) von weniger als 10, gemessen nach dem Verfahren E313, YI(E313-96) (D65) der American Society for Testing and Materials (ASTM), beibehält, wenn sie 24 Wochen lang bei 40 Grad C gelagert wird.

**11.** Topische Formulierung nach einem der Ansprüche 1 bis 10, wobei die topische Formulierung eine weiße Farbe mit einem Vergilbungsindex von weniger als 6, gemessen nach dem Verfahren E313, YI(E313-96) (D65) der American Society for Testing and Materials (ASTM), beibehält, wenn sie 16 Wochen lang bei 40 Grad C gelagert wird.

**12.** Topische Formulierung nach einem der Ansprüche 1 bis 10, wobei die topische Formulierung einen Gelbwert aufweist, der um nicht mehr als 8 ansteigt, gemessen nach dem Verfahren E313, YI(E313-96) (D65) der American Society for Testing and Materials (ASTM), über 24 Wochen bei 40 Grad C.

**13.** Topische Formulierung nach einem der Ansprüche 1 bis 12, wobei die weiße Vaseline nicht mehr als 7 ppm BHT einschließt.

**14.** Topische Formulierung nach Anspruch 1, ferner umfassend:

Tocopherol in einer Menge von weniger als 6,5 ppm;
Polyethylenglykol 400 in einer Menge von 10 Gew.-%;
Diethylenglykolmonoethylether in einer Menge von 15 Gew.-%;
Mineralöl in einer Menge von 5 Gew.-%;
ein emulgierendes Wachs in einer Menge von 10 Gew.-%; und
ein antimikrobielles Mittel 2-Phenoxyethanol in einer Menge von 1 Gew.-% umfasst;
wobei die topische Formulierung frei von BHT ist und dadurch einen Vergilbungsindex von weniger als 6 beibehält, wenn sie 16 Wochen lang bei 40 Grad C gelagert wird, und nicht mehr als 10, wenn sie 24 Wochen lang bei 40 Grad C gelagert wird, gemessen nach dem Verfahren E313, YI(E313-96) (D65) der American Society for Testing and Materials (ASTM).

**15.** Topische Formulierung nach einem der Ansprüche 1 bis 14 zur Verwendung bei der Behandlung einer dermatologischen Erkrankung, ausgewählt aus der Gruppe, bestehend aus Psoriasis, atopischer Dermatitis, atopischem Ekzem, chronischem Handekzem, Utikaria, Vitiligo, kutanem Lupus und Alopecia areata.

## Revendications

**1.** Formulation topique comprenant :

un inhibiteur de JAK dans une émulsion huile dans eau ;
de la vaseline blanche en une quantité de 10 % en poids ;
moins de 0,7 ppm de butylhydroxytoluène (BHT) en poids ;
de l'alcool oléique en une quantité de 2 % en poids ; et
un agent antimicrobien.

**2.** Formulation topique selon la revendication 1, dans laquelle l'inhibiteur de JAK est un inhibiteur de JAK1.

**3.** Formulation topique selon la revendication 1, dans laquelle l'inhibiteur de JAK est un inhibiteur de TYK2/JAK1.

**4.** Formulation topique selon la revendication 1, dans laquelle l'inhibiteur de JAK comprend la ((S)-2,2-difluorocyclopropyl)-((1R,5S)-3-(2-((1-méthyl-1H-pyrazol-4-yl)amino)pyrimidin-4-yl)-3,8-diazabicyclo[3.2.1]-octan-8-yl)méthanone.

**5.** Formulation topique selon l'une quelconque des revendications 1 à 4, dans laquelle l'inhibiteur de JAK est présent en

une quantité de 1 % à 3 % en poids.

6. Formulation topique selon l'une quelconque des revendications 1 à 5, comprenant du tocophérol en une quantité inférieure à 6,5 ppm.

7. Formulation topique selon l'une quelconque des revendications 1 à 6, comprenant du polyéthylène glycol 400 en une quantité de 10 % en poids.

8. Formulation topique selon l'une quelconque des revendications 1 à 7, comprenant de l'éther monoéthylique de diéthylène glycol en une quantité de 15 % en poids et éventuellement, de la cire émulsifiante en une quantité de 10 % en poids.

9. Formulation topique selon l'une quelconque des revendications 1 à 8, dans laquelle l'agent antimicrobien comprend du 2-phénoxyéthanol en une quantité de 1 % en poids.

10. Formulation topique selon l'une quelconque des revendications 1 à 9, dans laquelle la formulation topique conserve une couleur blanche ayant une valeur d'indice de jaunissement (YI) inférieure à 10, mesurée par la méthode E313 de l'American Society for Testing and Materials (ASTM), YI(E313-96) (D65), lorsqu'elle est stockée pendant 24 semaines à 40 degrés C.

11. Formulation topique selon l'une quelconque des revendications 1 à 10, dans laquelle la formulation topique conserve une couleur blanche ayant une valeur d'indice de jaunissement inférieure à 6, mesurée par la méthode E313 de l'American Society for Testing and Materials (ASTM), YI(E313-96) (D65), lorsqu'elle est stockée pendant 16 semaines à 40 degrés C.

12. Formulation topique selon l'une quelconque des revendications 1 à 10, dans laquelle la formulation topique a une valeur d'indice de jaunissement qui n'augmente pas de plus de 8, mesurée par la méthode E313 de l'American Society for Testing and Materials (ASTM), YI(E313-96) (D65), sur 24 semaines à 40 degrés C.

13. Formulation topique selon l'une quelconque des revendications 1 à 12, dans laquelle la vaseline blanche ne comprend pas plus de 7 ppm de BHT.

14. Formulation topique selon la revendication 1, comprenant en outre :

du tocophérol en une quantité inférieure à 6,5 ppm ;
du polyéthylène glycol 400 en une quantité de 10 % en poids ;
de l'éther monoéthylique de diéthylène glycol en une quantité de 15 % en poids ;
une huile minérale en une quantité de 5 % en poids ;
une cire émulsifiante en une quantité de 10 % en poids ; et
un agent antimicrobien comprend du 2-phénoxyéthanol en une quantité de 1 % en poids ; dans laquelle la formulation topique est exempte de BHT et maintient ainsi un indice de jaunissement inférieur à 6 lorsqu'elle est stockée à 40 degrés C pendant 16 semaines et de pas plus de 10 lorsqu'elle est stockée pendant 24 semaines à 40 degrés C, mesuré par la méthode E313 de l'American Society for Testing and Materials (ASTM), YI(E313-96) (D65).

15. Formulation topique selon l'une quelconque des revendications 1 à 14 destinée à être utilisée dans le traitement d'une affection dermatologique est choisie dans le groupe constitué par le psoriasis, la dermatite atopique, l'eczéma atopique, l'eczéma chronique des mains, l'urticaire, le vitiligo, le lupus cutané et l'alopécie areata.

FIG. 1

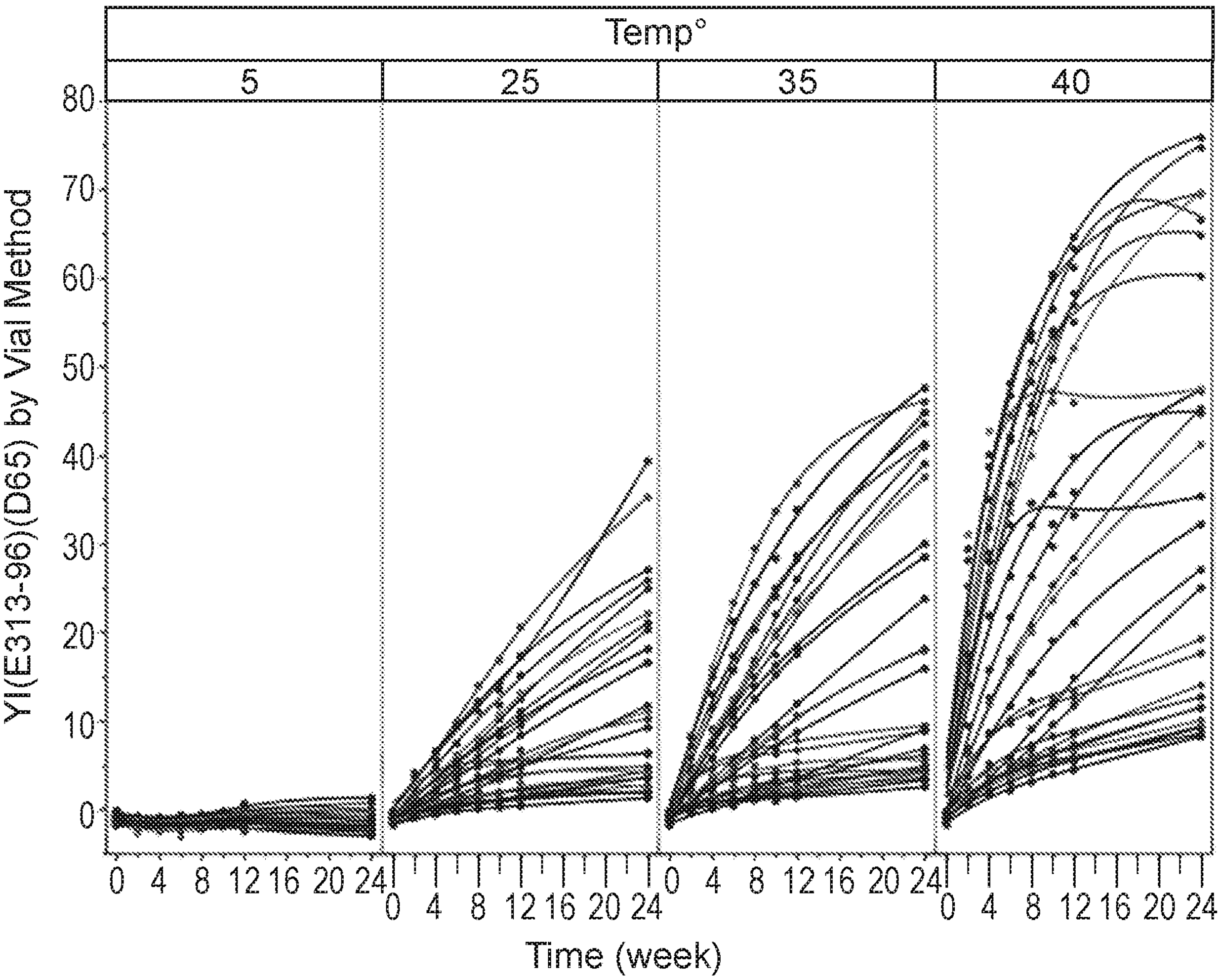
Temp°
5
25
35
40
80
70
60
50
40
30
20
10
0
YI(E313-96)(D65) by Vial Method
0 4 8 12 16 20 24
Time (week)

FIG. 2

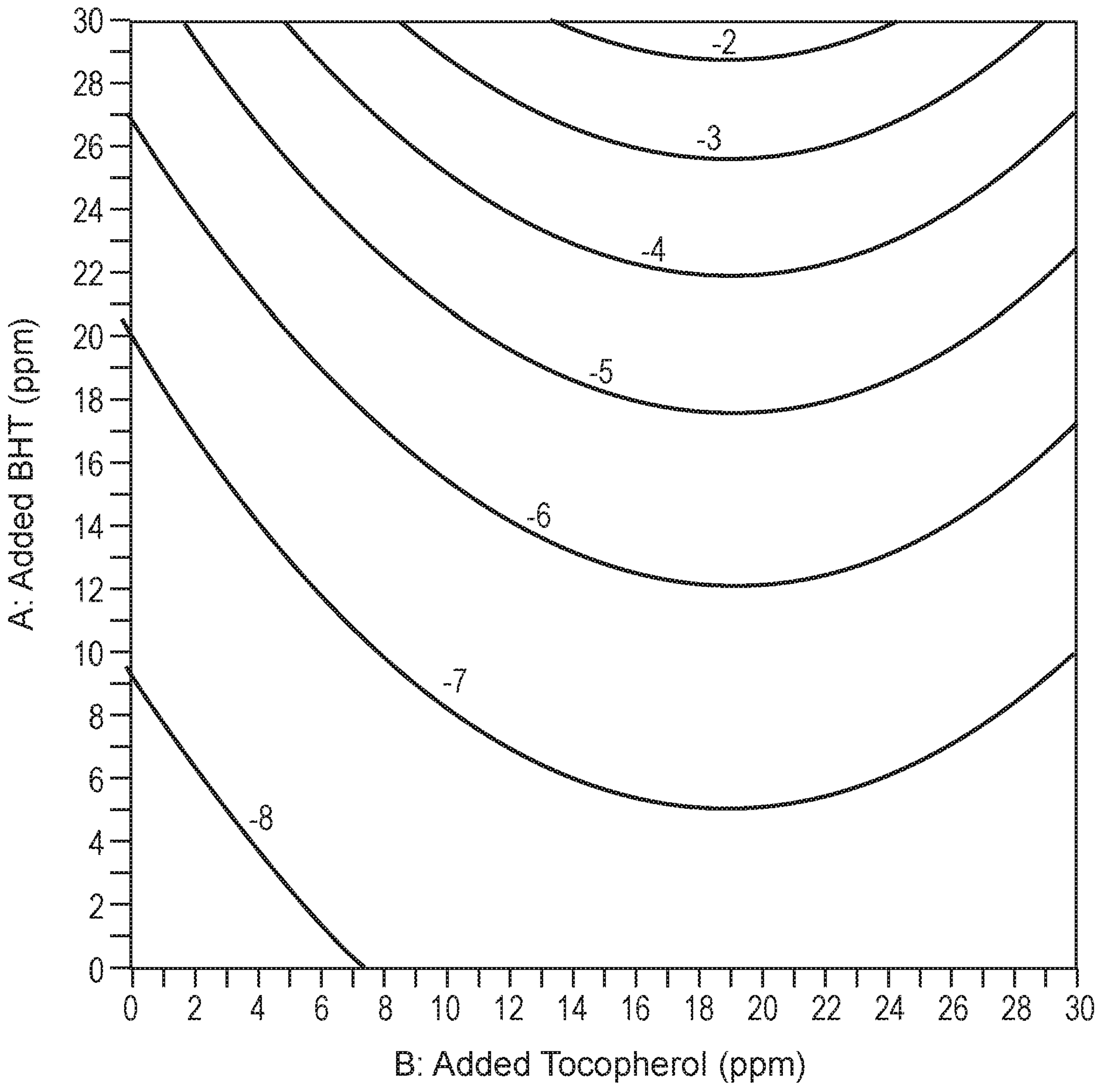

-2
-3
-4
-5
-6
-7
-8
30
28
26
24
22
20
18
16
14
12
10
8
6
4
2
0
A: Added BHT (ppm)
0
2
4
6
8
10
12
14
16
18
20
22
24
26
28
30
B: Added Tocopherol (ppm)

FIG. 3

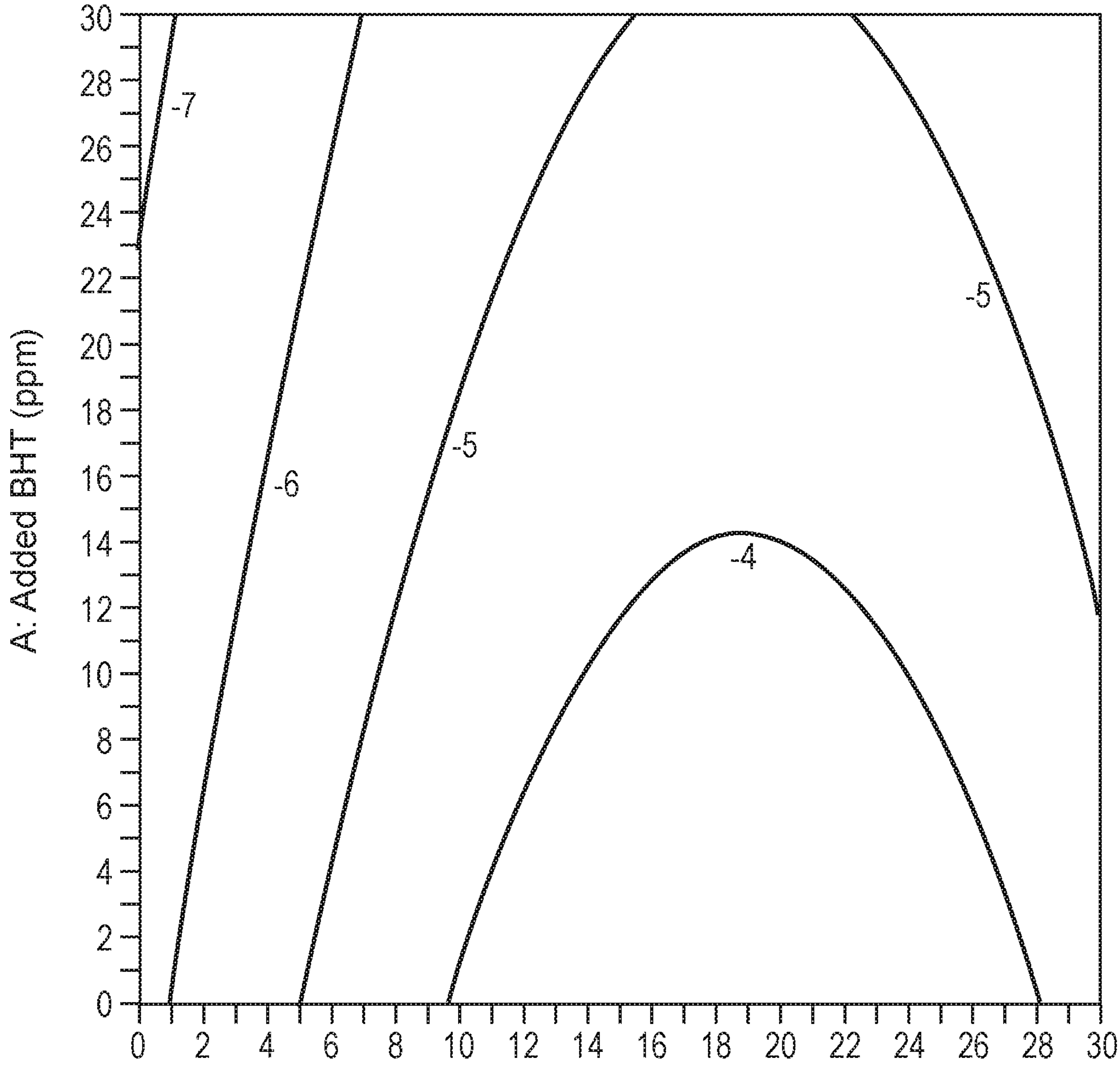
A: Added BHT (ppm)
0
2
4
6
8
10
12
14
16
18
20
22
24
26
28
30
B: Added Tocopherol (ppm)
-7
-6
-5
-5
-4

FIG. 4

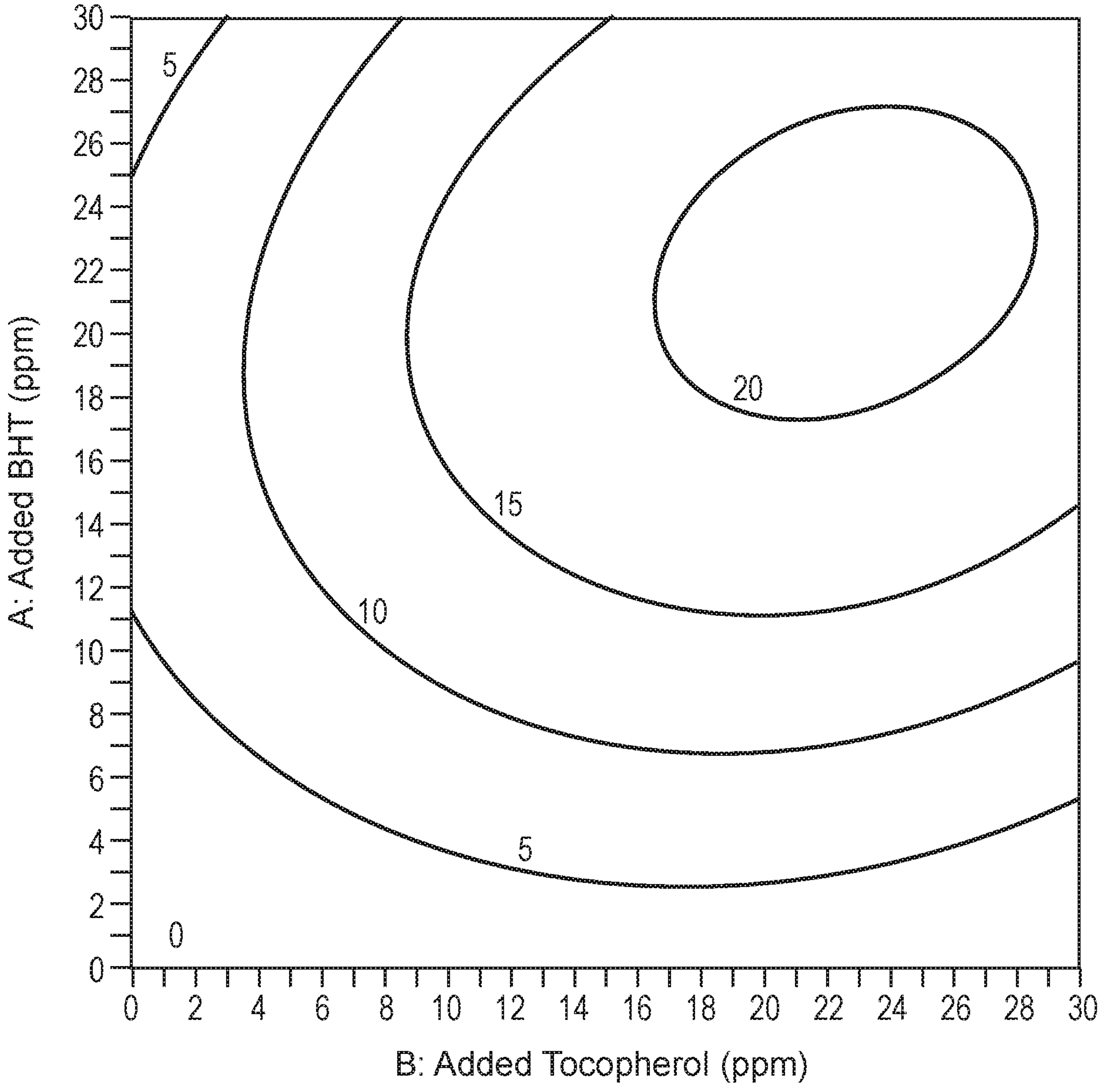

30
28
26
24
22
20
18
16
14
12
10
8
6
4
2
0
A: Added BHT (ppm)
0
2
4
6
8
10
12
14
16
18
20
22
24
26
28
30
B: Added Tocopherol (ppm)
5
20
15
10
5
0

FIG. 5

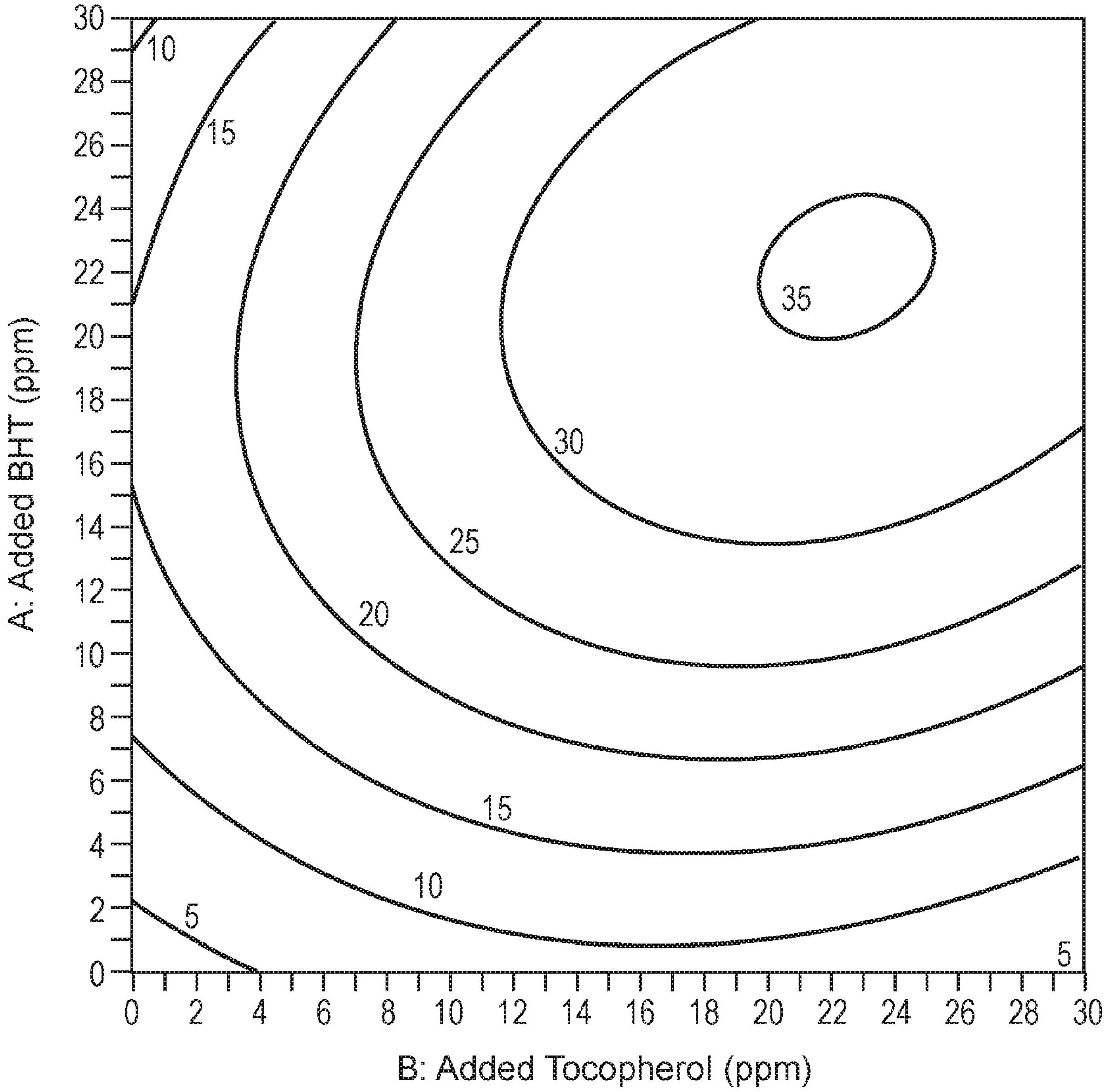
A: Added BHT (ppm)
B: Added Tocopherol (ppm)
0
2
4
6
8
10
12
14
16
18
20
22
24
26
28
30
5
10
15
20
25
30
35
10
15
5

FIG. 6

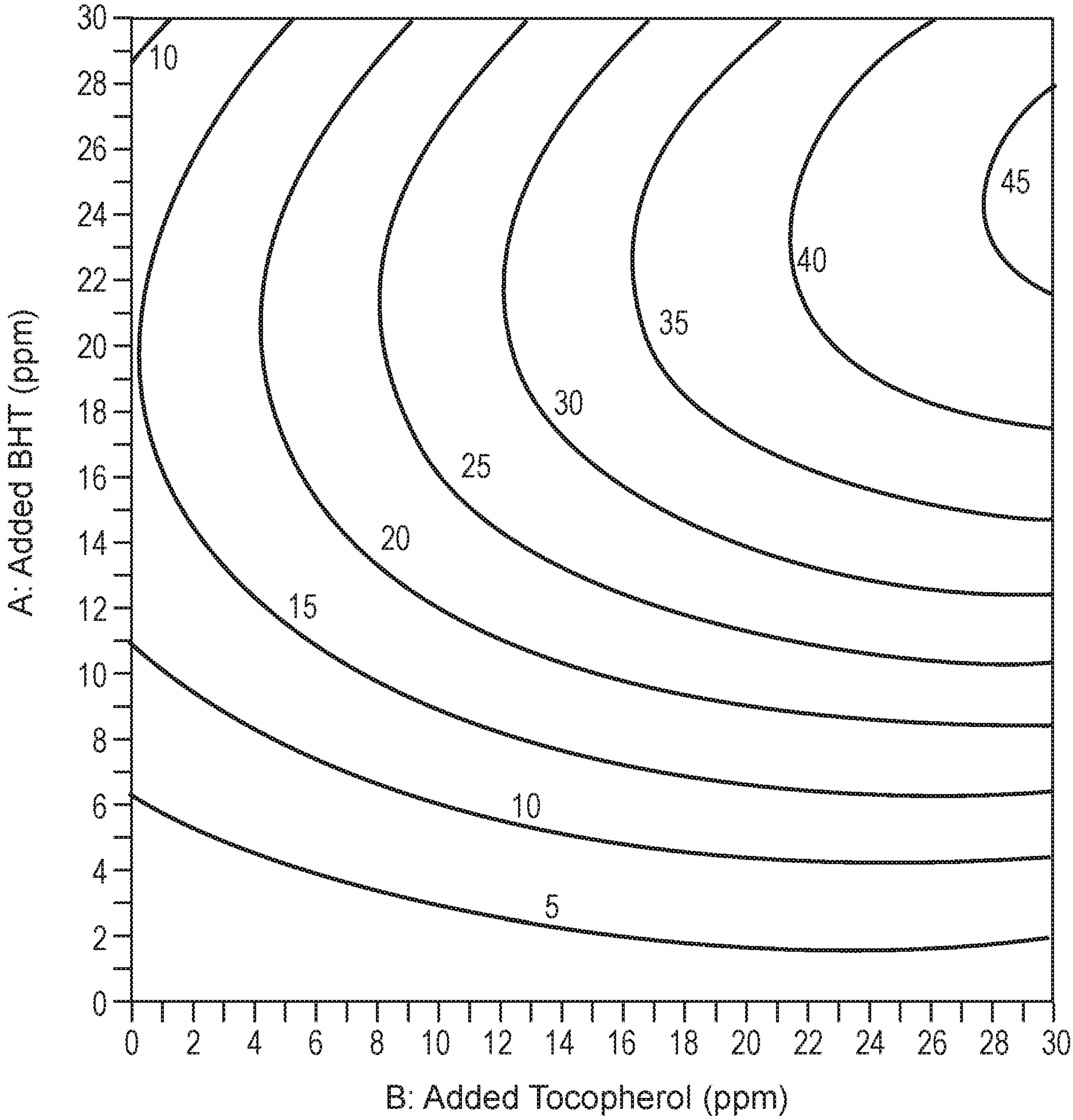
30
28
26
24
22
20
18
16
14
12
10
8
6
4
2
0
A: Added BHT (ppm)
0 2 4 6 8 10 12 14 16 18 20 22 24 26 28 30
B: Added Tocopherol (ppm)
10
45
40
35
30
25
20
15
10
5

FIG. 7

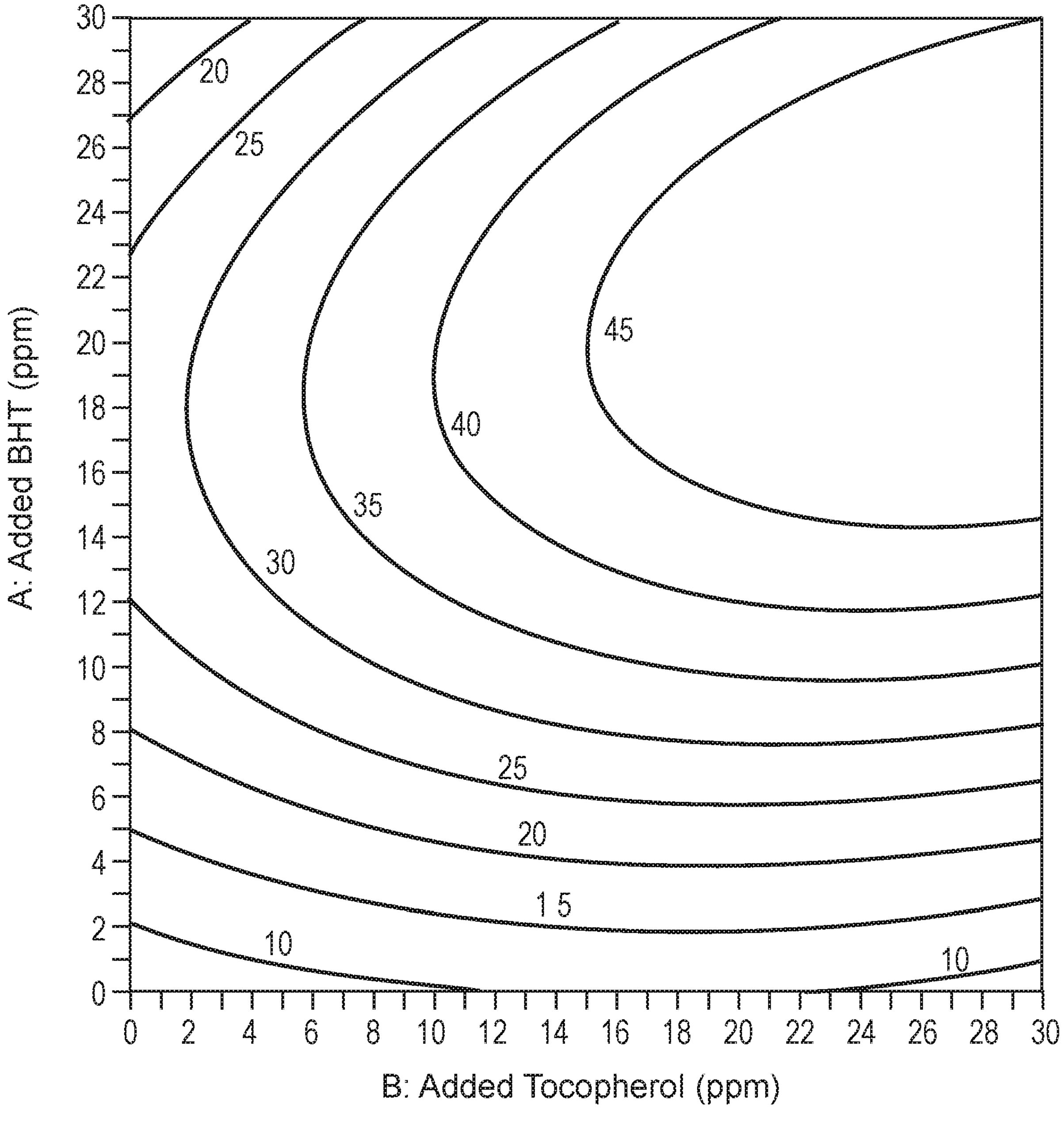
A: Added BHT (ppm)
B: Added Tocopherol (ppm)
0
2
4
6
8
10
12
14
16
18
20
22
24
26
28
30
20
25
30
35
40
45
25
20
1 5
10
10

FIG. 8

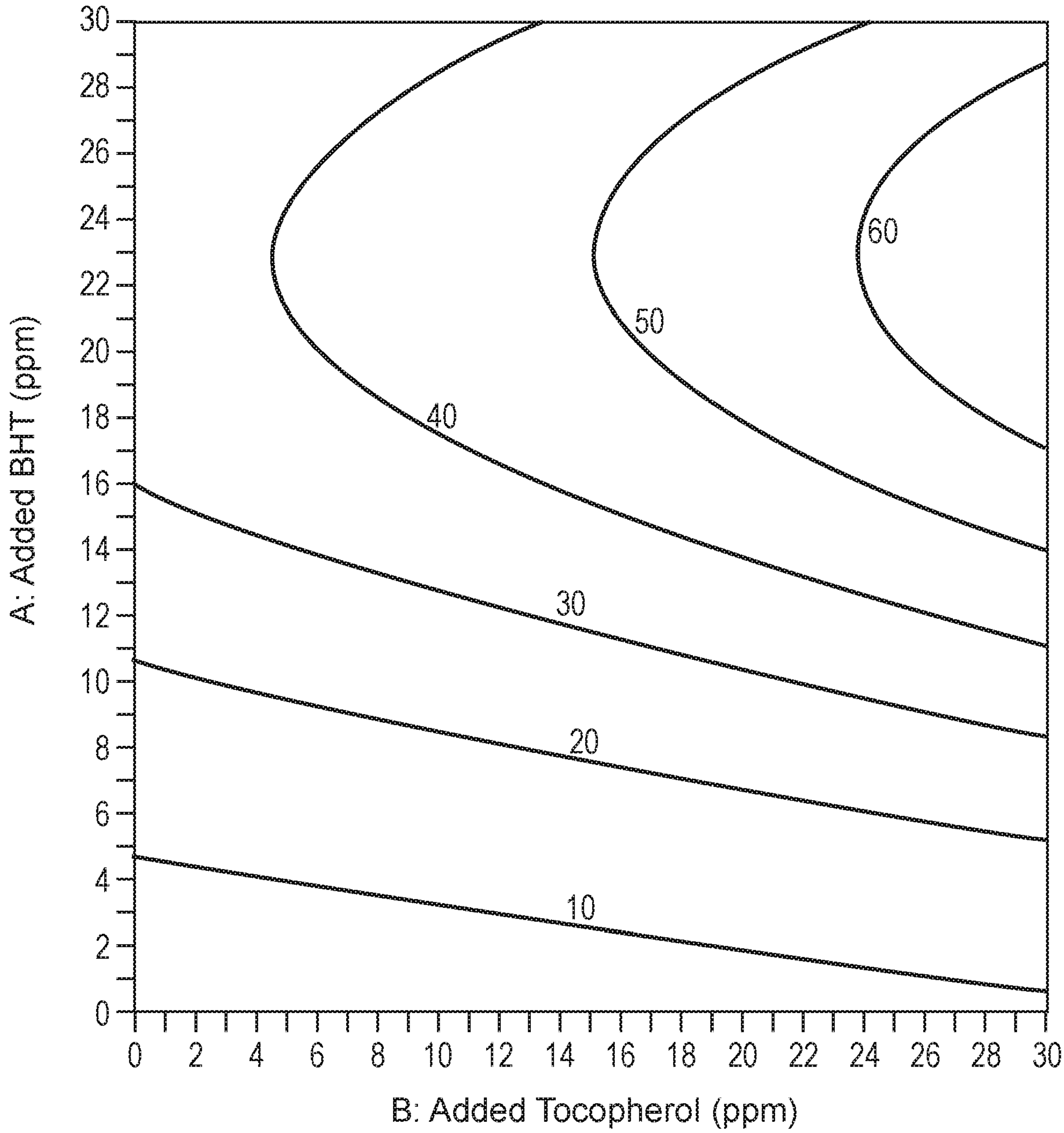
A: Added BHT (ppm)
B: Added Tocopherol (ppm)
0
2
4
6
8
10
12
14
16
18
20
22
24
26
28
30
10
20
30
40
50
60

FIG. 8A

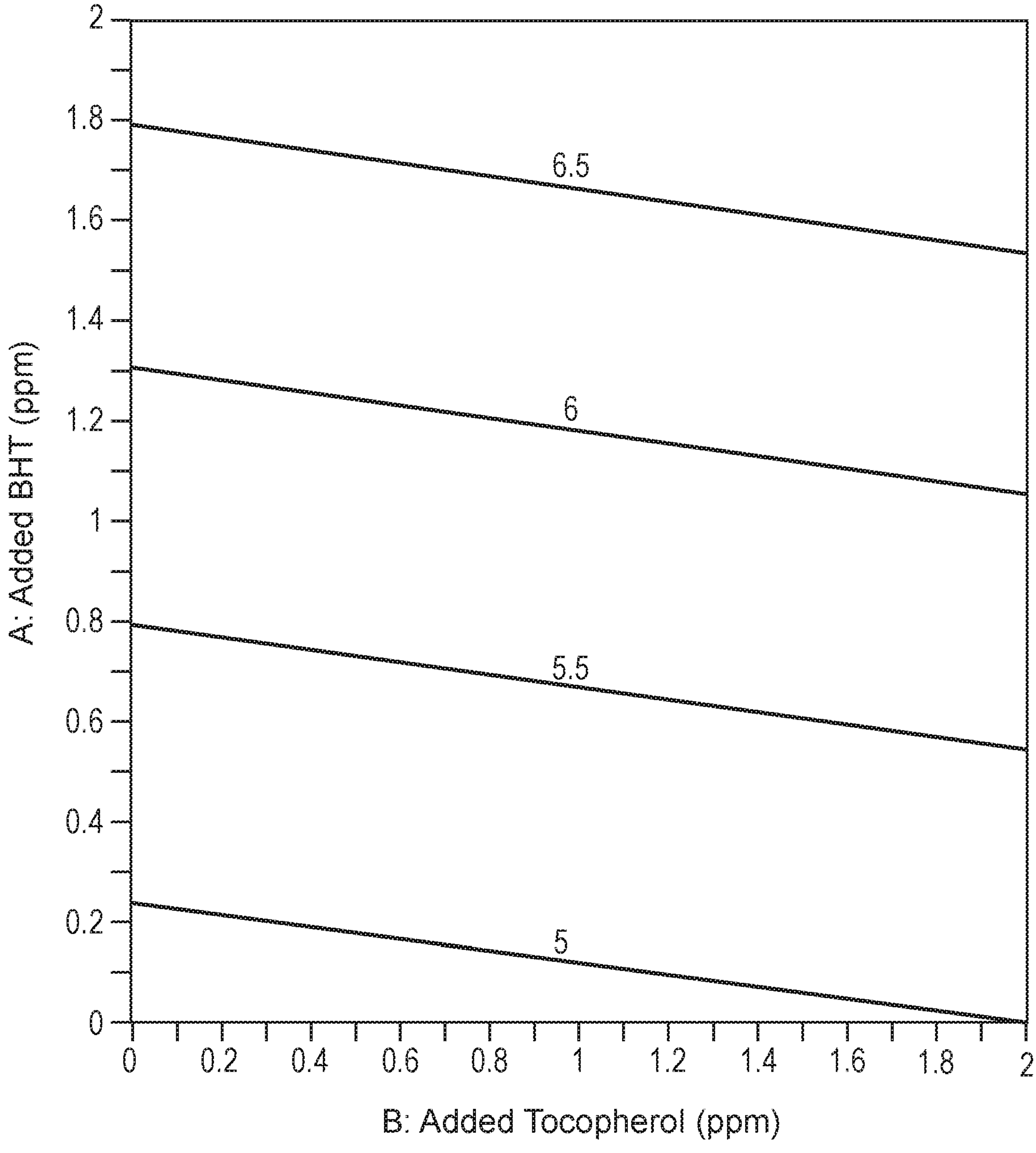
2
1.8
1.6
1.4
1.2
1
0.8
0.6
0.4
0.2
0
A: Added BHT (ppm)
6.5
6
5.5
5
0
0.2
0.4
0.6
0.8
1
1.2
1.4
1.6
1.8
2
B: Added Tocopherol (ppm)

# FIG. 9

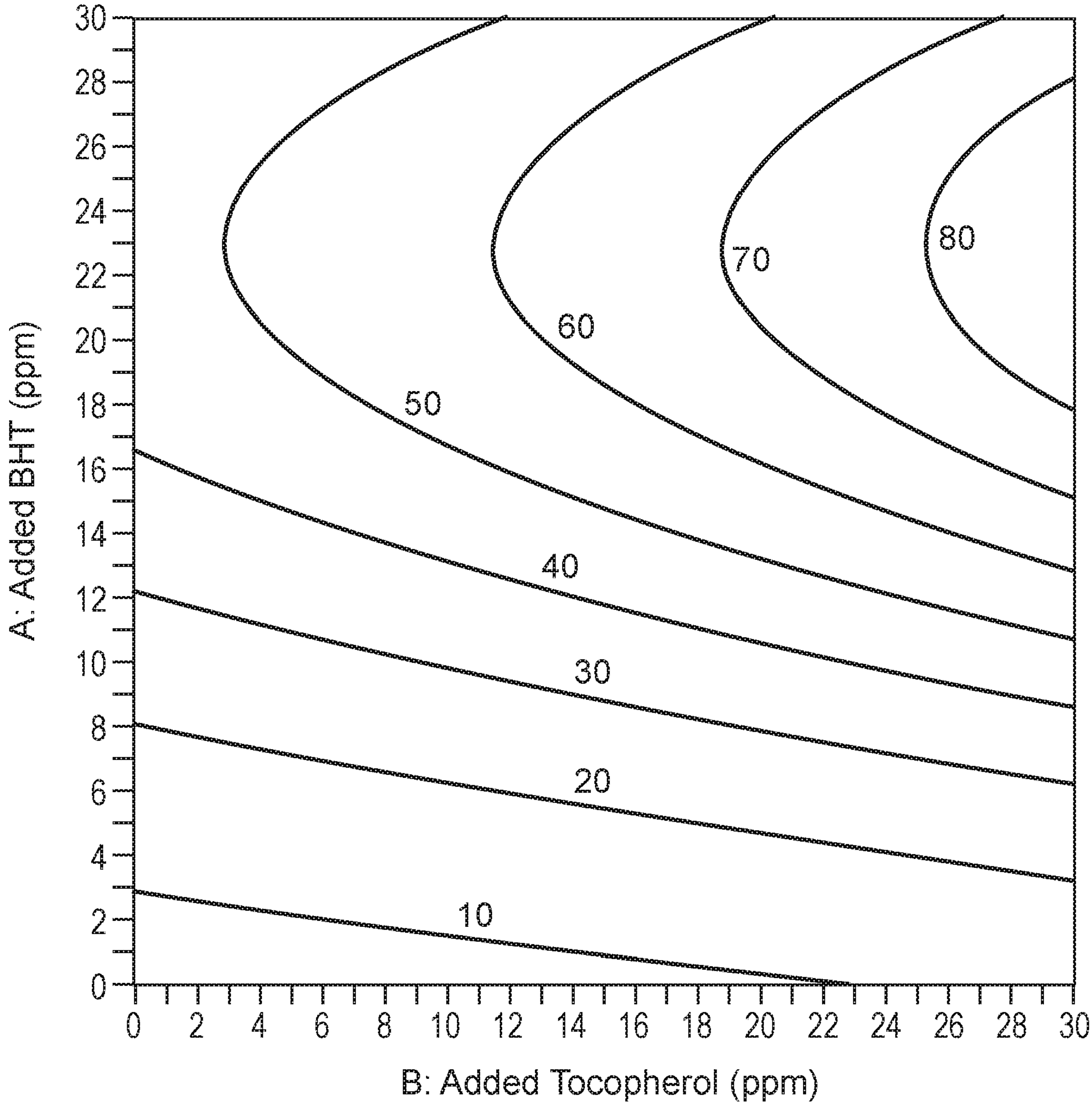
A: Added BHT (ppm)
B: Added Tocopherol (ppm)
0
2
4
6
8
10
12
14
16
18
20
22
24
26
28
30
10
20
30
40
50
60
70
80

# FIG. 10

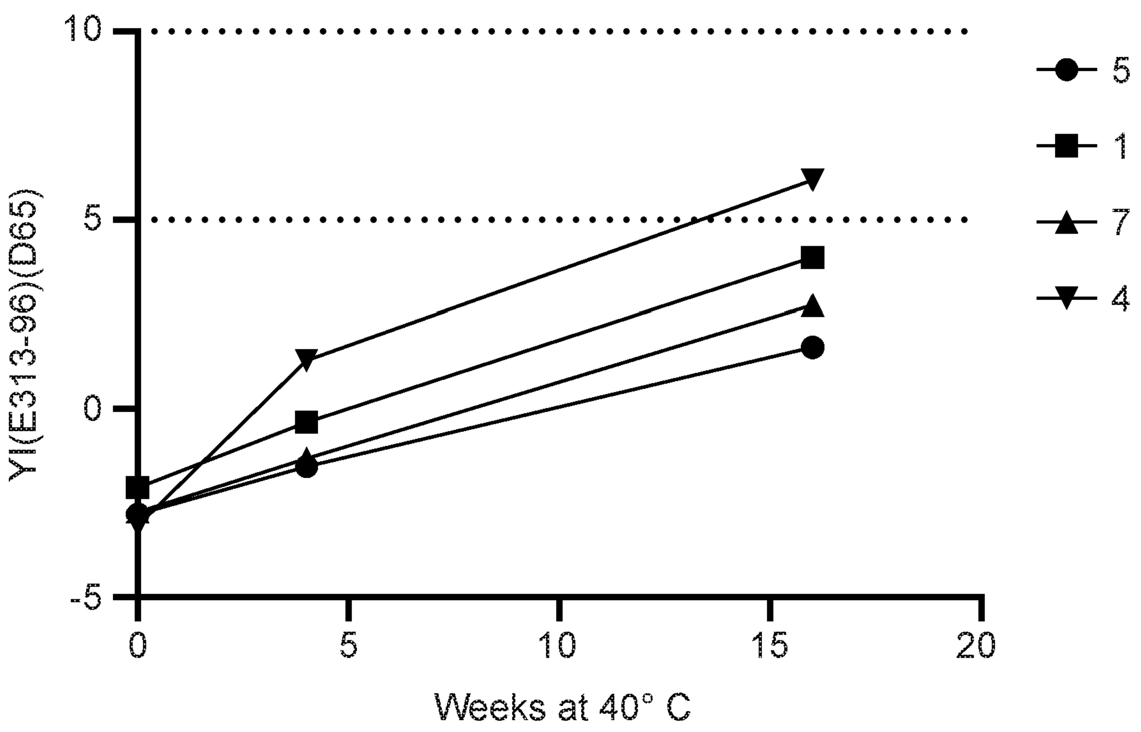

# FIG. 11

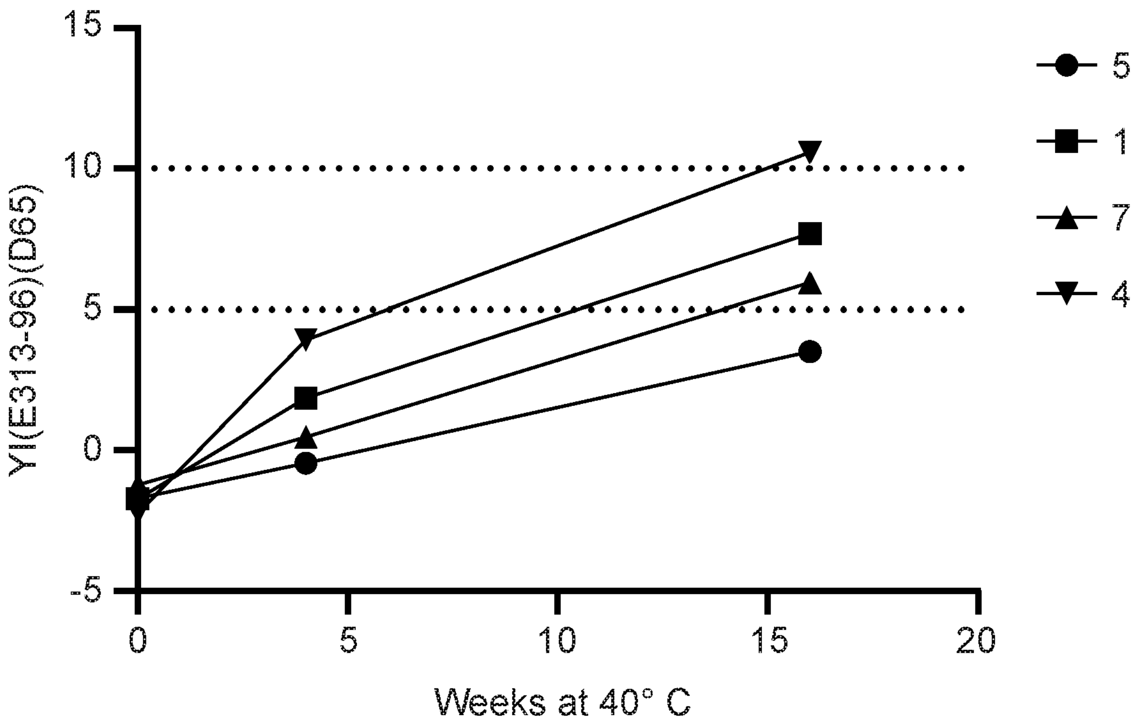

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10463675 B
- US 9663526 B
- US 63047606


### Non-patent literature cited in the description


- **B. C. FINNIN** ; **T. M. MORGAN**. *J. Pharm. Sci.*, 1999, vol. 88, 955-958